# EUROPEAN PATENT SPECIFICATION

(11) **EP 2 482 855 B1**
(45) Date of publication and mention of the grant of the patent: **27.12.2017**
(21) Application number: 10810996.8
(22) Date of filing: 29.09.2010
(51) Int. Cl.: A61K 47/60

(54) **OLIGOMER-CALCIMIMETIC CONJUGATES AND RELATED COMPOUNDS**
OLIGOMER-CALCIMIMETIKA-KONJUGATE UND ZUGEHÖRIGE VERBINDUNGEN
CONJUGUÉS OLIGOMÈRE-CALCIMIMÉTIQUE ET COMPOSÉS ASSOCIÉS

(30) Priority: 29.09.2009 US 246931 P
(43) Date of publication of application: 08.08.2012
(73) Proprietor: Nektar Therapeutics, San Francisco, CA 94158 (US)
(72) Inventor: RIGGS-SAUTHIER, Jennifer, Huntsville AL 35802 (US); CHENG, Lin, Sunnyvale CA 94085 (US); MARTIN, David, San Francisco CA 94107 (US)
(74) Representative: Boult Wade Tennant
(86) International application number: PCT/US2010/050761
(87) International publication number: WO 2011/056325

(56) References cited:
- WO-A2-2008/121386
- WO-A2-2009/151590
- US-A1- 2005 136 031
- MARCUS YONIT ET AL: "Turning low-molecular-weight drugs into prolonged acting prodrugs by reversible pegylation: A study with gentamicin", JOURNAL OF MEDICINAL CHEMISTRY, vol. 51, no. 14, July 2008 (2008-07), pages 4300-4305, XP002636000, ISSN: 0022-2623, DOI: 10.1021/JM8002558
- UREÑA PABLO ET AL: "Calcimimetic agents: review and perspectives.", KIDNEY INTERNATIONAL. SUPPLEMENT JUN 2003, vol. 63, no. 85, June 2003 (2003-06), pages S91-S96, XP002636001, ISSN: 0098-6577

## Description

### FIELD OF THE INVENTION

This invention comprises conjugates of a calcimimetic that possess certain advantages over the calcimimetic in unconjugated form. The conjugates described herein relate to and/or have application(s) in the fields of drug discovery, pharmacotherapy, physiology, organic chemistry and polymer chemistry.

### BACKGROUND OF THE INVENTION

Calcimimetics are small molecule drugs that mimic the action of calcium on tissues through allosteric activation of the calcium-sensing receptor that is expressed in various human organ tissues. Among other uses, calcimimetics are used to treat patients suffering from hyperparathyroidism, often caused by parathyroid cancers and chronic renal failure. A calcimimetic available commercially is (α*R*)-(-)-α-methyl-*N-*[3-[3-[trifluoromethylphenyl]propyl]-1-napthalenemethanamine or cinacalcet, sold as the SENSIPAR® brand of cinacalcet in North America and Australia (Amgen Inc., Thousand Oaks, CA) and the MIMPARA® brand of cinacalcet in Europe (Amgen Inc., Thousand Oaks, CA).

Briefly, calcimimetics are a class of orally active, small molecules that decrease the secretion of parathyroid hormone (PTH) by activating calcium receptors. The secretion of parathyroid hormone is normally regulated by the calcium-sensing receptor. Calcimimetics increase the sensitivity of this receptor to calcium, which inhibits the release of parathyroid hormone, thereby lowering parathyroid hormone levels within a few hours.

Despite the availability of cinacalcet as a representative member of the calcimimetics, its use in patients is associated with drawbacks. For example, cinacalcet is an inhibitor of CYP2D6, which can increase the blood concentration of drugs metabolized by CYP2D6. Thus, co-administration of cinacalcet with other drugs may cause deleterious drug-drug interactions. In addition, cinacalcet is typically administered at least once daily, whereas a less frequent dose (e.g., once a week dosing) would allow for a more convenient regimen. Further, a calcimimetic with a half-life longer than cinacalcet's 30 to 40 hours would avoid potential hypocalcemia and other risks associated with a reduced Cmax and/or greater time away from the Cmax. Reduction of one or more of these side effects from calcimimetics would enhance their desirability as therapeutic drugs. WO 2009/151590 A2 discloses CYP2D6 bioactive drugs conjugated to polyethylene glycol moieties.

WO 2008/121386 A2 discloses calcimimetic compounds for use in treating or preventing bowel disorders.

The present invention seeks to address these and other needs in the art.

### SUMMARY OF THE INVENTION

In one or more embodiments of the invention, a compound is provided, the compound comprising cinacalcet covalently attached via a releasable linkage to a poly(alkylene oxide) oligomer, wherein the linkage contains one or more amino acids, and pharmaceutically acceptable acid addition salts or complexes thereof. Compositions of enantiomers are also contemplated wherein the composition comprises one enantiomer in an amount greater than the other (e.g., one enantiomer is substantially present in the composition and the other enantiomer is substantially absent in the composition), wherein R enantiomers are preferred. Subject-matter which is not encompassed by the scope of the claims does not form part of the invention.

There are described compounds having the following structure: 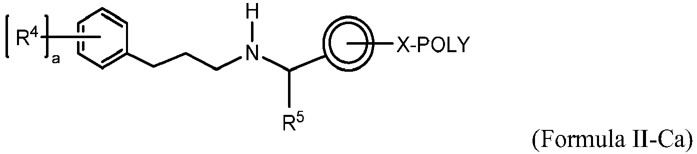 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

There are also described compounds having the following structure: 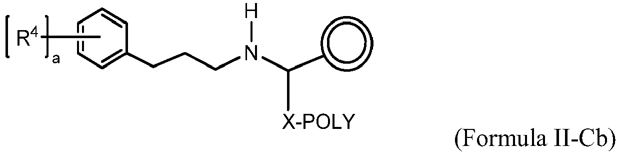 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
where "lower alkyl" is selected from a group consisting of 1 to 6 carbon atoms;
X is a spacer moiety (or "linkage"); and
POLY is a water-soluble, non-peptidic oligomer.

There are also described compounds having the following structure: 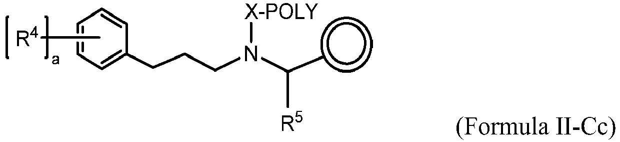 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

There are also described compounds having the following structure: 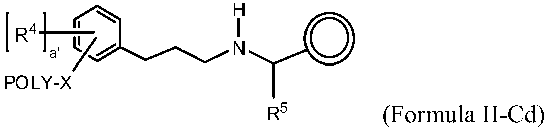 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a') is an integer from 1 to 4;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

The "calcimimetic residue" is a compound having a structure of a calcimimetic that is altered by the presence of one or more bonds, which bonds serve to attach (either directly or indirectly) one or more water-soluble, non-peptidic oligomers.

It is disclosed that any compound that is a calcimimetic can be used as a calcimimetic. Preferred calcimimetics are described in U.S. Patent No. 6,011,068.

Exemplary calcimimetic moieties have a structure encompassed by Formula I: 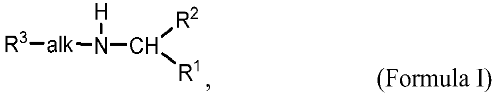 wherein:
alk is selected from the group consisting of n-propylene, 2,4-butylene and 1,3-butylene;
R¹ is selected from the group consisting of lower alkyl of from 1 to 3 carbon atoms and lower haloalkyl of from 1 to 3 carbon atoms substituted with from 1 to 7 halogen atoms; and
R² and R³ are independently selected monocyclic or bicyclic carbocyclic aryl or cycloalkyl groups, having 5- to 7-membered rings optionally substituted with 1 to 5 substituents independently selected from the group consisting of: OCF₃, lower alkyl of 1 to 3 carbon atoms, lower haloalkyl of 1 to 3 carbon atoms substituted with 1 to 7 halogen atoms, lower alkoxy of 1 to 3 carbon atoms, halogen, nitro, amino, alkylamino, amido, lower alkylamido of 1 to 3 carbon atoms, cyano, hydroxy, acyl of 2 to 4 carbon atoms, lower hydroxyalkyl of 1 to 3 carbon atoms, and lower thioalkyl of 1 to 3 carbon atoms; and
optionally, if R₂ is phenyl, then said phenyl R₂ has at least one substituent that is not 4-OH-phenyl.

Disclosed are calcimimetics that are encompassed by the following formula: 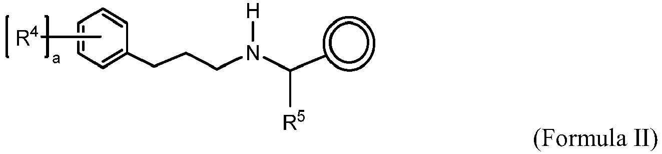 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl); and
where "lower alkyl" is selected from a group consisting of 1 to 6 carbon atoms.

In one or more embodiments of the invention, a composition is provided, the composition comprising a compound of the invention, and optionally, a pharmaceutically acceptable excipient.

There is described a dosage form comprising a compound comprising a calcimimetic residue covalently attached via a stable or releasable linkage to a water-soluble, non-peptidic oligomer, wherein the compound is present in a dosage form.

There is described a method comprising covalently attaching a water-soluble, non-peptidic oligomer to a calcimimetic.

There is described a method comprising administering a compound comprising a calcimimetic residue covalently attached via a stable or releasable linkage to a water-soluble, non-peptidic oligomer.

These and other objects, aspects, embodiments and features of the invention will become more fully apparent to one of ordinary skill in the art when read in conjunction with the following detailed description.

### BRIEF DESCRIPTION OF THE FIGURES

**FIG. 1A** and **FIG. 1B** are each graphs showing plasma concentration-time profiles for exemplary compounds and released cinacalcet from exemplary compounds, as further described in Example 14.
**FIG. 2** is a graph showing plasma concentration-time profiles for exemplary compounds and released cinacalcet from the exemplary compounds, as further described in Example 14.
**FIG. 3** is a graph showing plasma concentration-time profiles for exemplary compounds and released cinacalcet from the exemplary compounds, as further described in Example 14.
**FIG. 4** is a graph showing plasma concentration-time profiles for exemplary compounds and released cinacalcet from the exemplary compound, as further described in Example 14.
**FIG. 5** is a graph showing plasma concentration-time profiles for exemplary compounds and released cinacalcet from the exemplary compound, as further described in Example 14.
**FIG. 6** is a graph of rat plasma PTH levels following administration of cinacalcet (10mg/kg, po) and exemplary compounds (1mg/kg, IV), as further described in Example 17.
**FIG. 7** is a graph of rat plasma PTH levels following administration of cinacalcet (10mg/kg, po) and exemplary compounds (1mg/kg, IV), as further described in Example 17.
**FIG. 8** is a graph of rat plasma PTH levels following administration of cinacalcet (10mg/kg, po) and exemplary compounds (1mg/kg, IV), as further described in Example 17.
**FIG. 9** is a graph of rat plasma PTH levels following administration of cinacalcet (10mg/kg, po) and exemplary compounds (1mg/kg, IV), as further described in Example 17.

### DETAILED DESCRIPTION OF THE INVENTION

As used in this specification, the singular forms "a," "an," and "the" include plural referents unless the context clearly dictates otherwise.

In describing and claiming the present invention, the following terminology will be used in accordance with the definitions described below.

"Water soluble, non-peptidic oligomer" indicates an oligomer that is at least 35% (by weight) soluble, preferably greater than 70% (by weight), and more preferably greater than 95% (by weight) soluble, in water at room temperature. Typically, an unfiltered aqueous preparation of a "water-soluble" oligomer transmits at least 75%, more preferably at least 95%, of the amount of light transmitted by the same solution after filtering. It is most preferred, however, that the water-soluble oligomer is at least 95% (by weight) soluble in water or completely soluble in water. With respect to being "non-peptidic," an oligomer is non-peptidic when it has less than 35% (by weight) of amino acid residues.

The terms "monomer," "monomeric subunit" and "monomeric unit" are used interchangeably herein and refer to one of the basic structural units of a polymer or oligomer. In the case of a homo-oligomer, a single repeating structural unit forms the oligomer. In the case of a co-oligomer, two or more structural units are repeated -- either in a pattern or randomly -- to form the oligomer. Preferred oligomers used in connection with the present invention are homo-oligomers. The water-soluble, non-peptidic oligomer typically comprises one or more monomers serially attached to form a chain of monomers. The oligomer can be formed from a single monomer type (i.e., is homo-oligomeric) or two or three monomer types (i.e., is co-oligomeric).

An "oligomer" is a molecule possessing from about 1 to about 30 monomers. Specific oligomers for use in the invention include those having a variety of geometries such as linear, branched, or forked, to be described in greater detail below.

"PEG" or "polyethylene glycol," as used herein, is meant to encompass any water-soluble poly(ethylene oxide). Unless otherwise indicated, a "PEG oligomer" or an oligoethylene glycol is one in which substantially all (preferably all) monomeric subunits are ethylene oxide subunits, though the oligomer may contain distinct end capping moieties or functional groups, e.g., for conjugation. PEG oligomers for use in the present invention will comprise one of the two following structures: "-(CH₂CH₂O)ₙ-" or "-(CH₂CH₂O)ₙ₋₁CH₂CH₂-," depending upon whether or not the terminal oxygen(s) has been displaced, e.g., during a synthetic transformation. As stated above, for the PEG oligomers, the variable (n) ranges from 1 to 30, and the terminal groups and architecture of the overall PEG can vary. When PEG further comprises a functional group, A, for linking to, e.g., a small molecule drug, the functional group when covalently attached to a PEG oligomer does not result in formation of (i) an oxygen-oxygen bond (-O-O-, a peroxide linkage), or (ii) a nitrogen-oxygen bond (N-O, O-N).

The terms "end-capped" or "terminally capped" are interchangeably used herein to refer to a terminal or endpoint of a polymer having an end-capping moiety. Typically, although not necessarily, the end-capping moiety comprises a hydroxy or C₁₋₂₀ alkoxy group. Thus, examples of end-capping moieties include alkoxy (e.g., methoxy, ethoxy and benzyloxy), as well as aryl, heteroaryl, cyclo and heterocyclo. In addition, saturated, unsaturated, substituted and unsubstituted forms of each of the foregoing are envisioned. Moreover, the end-capping group can also be a silane. The end-capping group can also advantageously comprise a detectable label. When the polymer has an end-capping group comprising a detectable label, the amount or location of the polymer and/or the moiety (e.g., active agent) of interest to which the polymer is coupled, can be determined by using a suitable detector. Such labels include, fluorescers, chemiluminescers, moieties used in enzyme labeling, colorimetric moieties (e.g., dyes), metal ions and radioactive moieties. Suitable detectors include photometers, films and spectrometers. In addition, the end-capping group may contain a targeting moiety.

The term "targeting moiety" is used herein to refer to a molecular structure that helps conjugates to localize to a targeting area, e.g., help enter a cell, or bind a receptor. Preferably, the targeting moiety comprises of vitamin, antibody, antigen, receptor, DNA, RNA, sialyl Lewis X antigen, hyaluronic acid, sugars, cell specific lectins, steroid or steroid derivative, RGD peptide, ligand for a cell surface receptor, serum component, or combinatorial molecule directed against various intra- or extracellular receptors. The targeting moiety may also comprise a lipid or a phospholipid. Exemplary phospholipids include, phosphatidylcholines, phospatidylserine, phospatidylinositol, phospatidylglycerol, and phospatidylethanolamine. These lipids may be in the form of micelles or liposomes. The targeting moiety may further comprise a detectable label or alternately a detectable label may serve as a targeting moiety. When the conjugate has a targeting group comprising a detectable label, the amount and/or distribution/location of the polymer and/or the moiety (e.g., active agent) to which the polymer is coupled can be determined by using a suitable detector. Such labels include, fluorescers, chemiluminescers, moieties used in enzyme labeling, colorimetric (e.g., dyes), metal ions, radioactive moieties, gold particles and quantum dots.

"Branched," in reference to the geometry or overall structure of an oligomer, refers to an oligomer having two or more polymer "arms" extending from a branch point.

"Forked," in reference to the geometry or overall structure of an oligomer, refers to an oligomer having two or more functional groups (typically through one or more atoms) extending from a branch point.

A "branch point" refers to a bifurcation point comprising one or more atoms at which an oligomer branches or forks from a linear structure into one or more additional arms.

The term "reactive" or "activated" refers to a functional group that reacts readily or at a practical rate under conventional conditions of organic synthesis. This is in contrast to those groups that either do not react or require strong catalysts or impractical reaction conditions in order to react (i.e., a "nonreactive" or "inert" group).

"Not readily reactive," with reference to a functional group present on a molecule in a reaction mixture, indicates that the group remains largely intact under conditions that are effective to produce a desired reaction in the reaction mixture.

A "protecting group" is a moiety that prevents or blocks reaction of a particular chemically reactive functional group in a molecule under certain reaction conditions. The protecting group may vary depending upon the type of chemically reactive group being protected as well as the reaction conditions to be employed and the presence of additional reactive or protecting groups in the molecule. Functional groups which may be protected include, by way of example, carboxylic acid groups, amino groups, hydroxyl groups, thiol groups and carbonyl groups. Representative protecting groups for carboxylic acids include esters (such as a *p*-methoxybenzyl ester), amides and hydrazides; for amino groups, carbamates (such as *tert*-butoxycarbonyl) and amides; for hydroxyl groups, ethers and esters; for thiol groups, thioethers and thioesters; for carbonyl groups, acetals and ketals. Such protecting groups are well-known to those skilled in the art and are described, for example, in T.W. Greene and G.M. Wuts, Protecting Groups in Organic Synthesis, Third Edition, Wiley, New York, 1999, and references cited therein.

A functional group in "protected form" refers to a functional group bearing a protecting group. As used herein, the term "functional group" or any synonym thereof encompasses protected forms thereof.

A "physiologically cleavable" or "hydrolyzable" or "degradable" bond is a relatively labile bond that reacts with water (i.e., is hydrolyzed) under physiological conditions. The tendency of a bond to hydrolyze in water may depend not only on the general type of linkage connecting two central atoms but also on the substituents attached to these central atoms. Appropriate hydrolytically unstable or weak linkages include carboxylate ester, phosphate ester, anhydrides, acetals, ketals, acyloxyalkyl ether, imines, orthoesters, peptides, oligonucleotides, thioesters, and carbonates.

An "enzymatically releasable linkage" means a linkage that is subject to degradation by one or more enzymes.

A "stable" linkage or bond refers to a chemical bond that is substantially stable in water, that is to say, does not undergo hydrolysis under physiological conditions to any appreciable extent over an extended period of time. Examples of hydrolytically stable linkages include the following: carbon-carbon bonds (e.g., in aliphatic chains), ethers, amides, urethanes and amines. Generally, a stable linkage is one that exhibits a rate of hydrolysis of less than about 1-2% per day under physiological conditions. Hydrolysis rates of representative chemical bonds can be found in most standard chemistry textbooks.

"Substantially" or "essentially" means nearly totally or completely, for instance, 95% or greater, more preferably 97% or greater, still more preferably 98% or greater, even more preferably 99% or greater, yet still more preferably 99.9% or greater, with 99.99% or greater being most preferred of some given quantity.

"Monodisperse" refers to an oligomer composition wherein substantially all of the oligomers in the composition have a well-defined, single molecular weight and defined number of monomers, as determined by chromatography or mass spectrometry. Monodisperse oligomer compositions are in one sense pure, that is, substantially having a single and definable number (as a whole number) of monomers rather than a large distribution. A monodisperse oligomer composition possesses a MW/Mn value of 1.0005 or less, and more preferably, a MW/Mn value of 1.0000. By extension, a composition comprised of monodisperse conjugates means that substantially all oligomers of all conjugates in the composition have a single and definable number (as a whole number) of monomers rather than a large distribution and would possess a MW/Mn value of 1.0005, and more preferably, a MW/Mn value of 1.0000 if the oligomer were not attached to the calcimimetic. A composition comprised of monodisperse conjugates may, however, include one or more nonconjugate substances such as solvents, reagents, excipients, and so forth.

"Bimodal," in reference to an oligomer composition, refers to an oligomer composition wherein substantially all oligomers in the composition have one of two definable and different numbers (as whole numbers) of monomers rather than a large distribution, and whose distribution of molecular weights, when plotted as a number fraction versus molecular weight, appears as two separate identifiable peaks. Preferably, for a bimodal oligomer composition as described herein, each peak is generally symmetric about its mean, although the size of the two peaks may differ. Ideally, the polydispersity index of each peak in the bimodal distribution, Mw/Mn, is 1.01 or less, more preferably 1.001 or less, and even more preferably 1.0005 or less, and most preferably a MW/Mn value of 1.0000. By extension, a composition comprised of bimodal conjugates means that substantially all oligomers of all conjugates in the composition have one of two definable and different numbers (as whole numbers) of monomers rather than a large distribution and would possess a MW/Mn value of 1.01 or less, more preferably 1.001 or less and even more preferably 1.0005 or less, and most preferably a MW/Mn value of 1.0000 if the oligomer were not attached to the calcimimetic. A composition comprised of bimodal conjugates may, however, include one or more nonconjugate substances such as solvents, reagents, excipients, and so forth.

A "calcimimetic" refers to an organic, inorganic, or organometallic compound having a molecular weight of less than about 1000 Daltons and having some degree of calcimimetic activity. By using known assays, one of ordinary skill in the art will be able to determine whether any given compound has calcimimetic activity. Among the activities of a calcimimetic, increasing the sensitivity of the calcium-sensing receptor to activation by extracellular calcium is exemplary.

A "biological membrane" is any membrane made of cells or tissues that serves as a barrier to at least some foreign entities or otherwise undesirable materials. As used herein a "biological membrane" includes those membranes that are associated with physiological protective barriers including, for example: the blood-brain barrier (BBB); the blood-cerebrospinal fluid barrier; the blood-placental barrier; the blood-milk barrier; the blood-testes barrier; and mucosal barriers including the vaginal mucosa, urethral mucosa, anal mucosa, buccal mucosa, sublingual mucosa, and rectal mucosa. Unless the context clearly dictates otherwise, the term "biological membrane" does not include those membranes associated with the middle gastro-intestinal tract (e.g., stomach and small intestines).

A "biological membrane crossing rate," provides a measure of a compound's ability to cross a biological membrane, such as the blood-brain barrier ("BBB"). A variety of methods may be used to assess transport of a molecule across any given biological membrane. Methods to assess the biological membrane crossing rate associated with any given biological barrier (e.g., the blood-cerebrospinal fluid barrier, the blood-placental barrier, the blood-milk barrier, the intestinal barrier, and so forth), are known, described herein and/or in the relevant literature, and/or may be determined by one of ordinary skill in the art.

"Alkyl" refers to a hydrocarbon chain, ranging from about 1 to 20 atoms in length. Such hydrocarbon chains are preferably but not necessarily saturated and may be branched or straight chain. Exemplary alkyl groups include methyl, ethyl, propyl, butyl, pentyl, 2-methylbutyl, 2-ethylpropyl and 3-methylpentyl. As used herein, "alkyl" includes cycloalkyl when three or more carbon atoms are referenced.

"Lower alkyl" refers to an alkyl group containing from 1 to 6 carbon atoms, and may be straight chain or branched, as exemplified by methyl, ethyl, n-butyl, i-butyl, t-butyl.

"Non-interfering substituents" are those groups that, when present in a molecule, are typically non-reactive with other functional groups contained within the molecule.

"Alkoxy" refers to an -O-R group, wherein R is alkyl or substituted alkyl, preferably C₁-C₂₀ alkyl (e.g., methoxy, ethoxy, propyloxy, benzyl, etc.), preferably C₁-C₇.

"Pharmaceutically acceptable excipient" or "pharmaceutically acceptable carrier" refers to a component that may be included in the compositions causing no significant adverse toxicological effects to a patient.

The term "aryl" means an aromatic group having up to 14 carbon atoms. Aryl groups include phenyl, naphthyl, biphenyl and phenanthrenyl, naphthacenyl. "Substituted phenyl" and "substituted aryl" denote a phenyl group and aryl group, respectively, substituted with one, two, three, four or five (e.g., 1-2, 1-3 or 1-4 substituents) chosen from halo (F, Cl, Br, I), hydroxy, hydroxy, cyano, nitro, alkyl (e.g., C₁₋₆ alkyl), alkoxy (e.g., C₁₋₆ alkoxy), benzyloxy, carboxy, aryl.

"Pharmacologically effective amount," "physiologically effective amount," and "therapeutically effective amount" are used interchangeably herein to mean the amount of a water-soluble oligomer-small molecule drug conjugate present in a composition that is needed to provide a desired level of active agent and/or conjugate in the bloodstream or in the target tissue. The precise amount may depend upon numerous factors, e.g., the particular active agent, the components and physical characteristics of the composition, intended patient population, patient considerations, and may readily be determined by one skilled in the art, based upon the information provided herein and available in the relevant literature.

A "difunctional" oligomer is an oligomer having two functional groups contained therein, typically at its termini. When the functional groups are the same, the oligomer is said to be homodifunctional. When the functional groups are different, the oligomer is said to be heterobifunctional.

A basic reactant or an acidic reactant described herein include neutral, charged, and any corresponding salt forms thereof.

The term "patient," refers to a living organism suffering from or prone to a condition that can be prevented or treated by administration of a conjugate as described herein, and includes both humans and animals.

"Optional" or "optionally" means that the subsequently described circumstance may but need not necessarily occur, so that the description includes instances where the circumstance occurs and instances where it does not.

As indicated above, the present invention is directed to a compound comprising cinacalcet covalently attached via a releasable linkage to a poly(alkylene oxide) oligomer, wherein the linkage contains one or more amino acids, and pharmaceutically acceptable acid addition salts or complexes thereof.

There is described a compound comprising a calicimimetic residue covalently attached via a stable or releasable linkage to a water-soluble, non-peptidic oligomer, and pharmaceutically acceptable acid addition salt or complexes thereof, wherein the calcimimetic has the following structure: 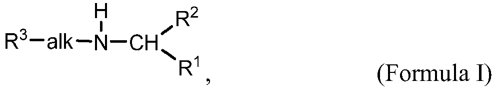 wherein:
alk is selected from the group consisting of n-propylene, 2,4-butylene and 1,3-butylene;
R¹ is selected from the group consisting of lower alkyl of from 1 to 3 carbon atoms and lower haloalkyl of from 1 to 3 carbon atoms substituted with from 1 to 7 halogen atoms; and
R² and R³ are independently selected monocyclic or bicyclic carbocyclic aryl or cycloalkyl groups, having 5- to 7-membered rings optionally substituted with 1 to 5 substituents independently selected from the group consisting of: OCF₃, lower alkyl of 1 to 3 carbon atoms, lower haloalkyl of 1 to 3 carbon atoms substituted with 1 to 7 halogen atoms, lower alkoxy of 1 to 3 carbon atoms, halogen, nitro, amino, alkylamino, amido, lower alkylamido of 1 to 3 carbon atoms, cyano, hydroxy, acyl of 2 to 4 carbon atoms, lower hydroxyalkyl of 1 to 3 carbon atoms, and lower thioalkyl of 1 to 3 carbon atoms; and
optionally, if R₂ is phenyl, then said phenyl R₂ has at least one substituent that is not 4-OH-phenyl.

Disclosed are calcimimetics that are encompassed by the following formula: 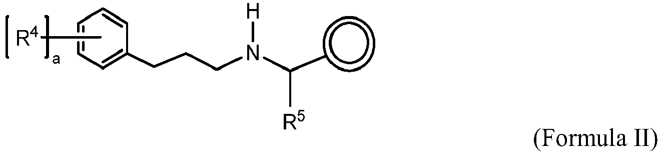 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl); and
where "lower alkyl" is selected from a group consisting of 1 to 6 carbon atoms.

Calcimimetic moieties encompassed by Formulae I and II and other calcimimetics are described in U.S. Patent Nos. 6,011,068 and 6,211,244. Approaches for preparing calcimimetics are described in U.S. Patent Nos. 7,563,930, 7,393,967, 7,250,533, 6,211,244, 6,011,068 5,648,541 and 4,966,988.

There are described compounds having the following structure: 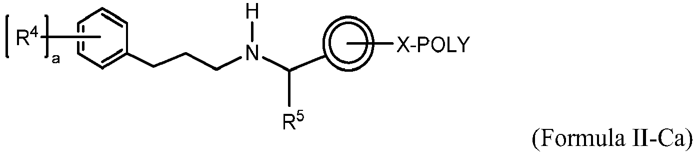 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

There are described compounds having the following structure: 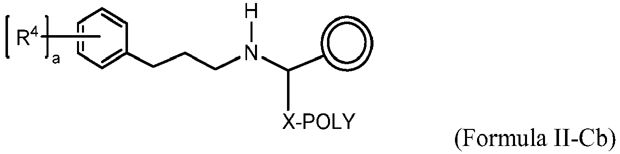 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
where "lower alkyl" is selected from a group consisting of 1 to 6 carbon atoms;
X is a spacer moiety (or "linkage"); and
POLY is a water-soluble, non-peptidic oligomer.

There are described compounds having the following structure: 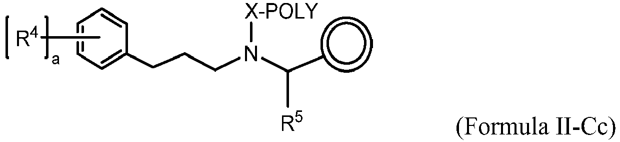 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a) is an integer from 1 to 5;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

There are described compounds having the following structure: 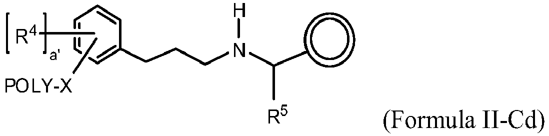 wherein:
⊚ is selected from the group consisting of 3-methoxyphenyl, 3-chlorophenyl and 1-naphthyl;
each R⁴ is independently selected from the group consisting of -H, -F, -Cl, -Br, -I, phenyl, -CF₃, -CF₂H, -CFH₂, lower alkyl (e.g., -CH₃), -O-lower alkyl (e.g., -OCH₃), -OCH₂CF₃, -OH, -CN, -NO₂, -C(O)-lower alkyl (e.g., -C(O)CH₃), -C(O)O-lower alkyl (e.g., -C(O)OCH₃), -C(O)NH-lower alkyl (e.g., -C(O)NH-CH₃), -C(O)N-lower alkyl₂ (e.g., -C(O)N(CH₃)₂), -OC(O)-lower alkyl (e.g., -OC(O)CH₃), and -NH-C(O)-lower alkyl (e.g., -NH-C(O)CH₃);
(a') is an integer from 1 to 4;
R⁵ is lower alkyl (e.g., methyl);
where "lower alkyl" is selected from a group consisting of 1 to 6 carbon atoms;
X is a spacer moiety (or "linkage"); and
POLY is a water-soluble, non-peptidic oligomer.

Use of oligomers (e.g., from a monodisperse or bimodal composition of oligomers, in contrast to relatively impure compositions) to form oligomer-containing compounds can advantageously alter certain properties associated with the corresponding small molecule drug. For instance, a compound of the invention, when administered by any of a number of suitable administration routes, such as parenteral, oral, transdermal, buccal, pulmonary, or nasal, exhibits altered metabolism at CYP2D6. Moreover, the compounds of the invention maintain a degree of bioactivity as well as bioavailability in comparison to the bioactivity and bioavailability of the compound free of all oligomers.

Assays for determining whether a given compound (regardless of whether the compound includes a water-soluble, non-peptidic oligomer or not) has calcimimetic activity are known and/or can be prepared by one of ordinary skill in the art.

Each of these (and other) calcimimetic moieties can be covalently attached (either directly or through one or more atoms) to a water-soluble and non-peptidic oligomer.

Exemplary molecular weights of small molecule drugs serving as a calcimimetic include molecular weights of: less than about 950; less than about 900; less than about 850; less than about 800; less than about 750; less than about 700; less than about 650; less than about 600; less than about 550; less than about 500; less than about 450; less than about 400; less than about 350; and less than about 300, Daltons.

The small molecule drug (i.e., calcimimetic) used in the invention is cinacalcet. This compound may be in a racemic mixture, or an optically active form, for example, a single optically active enantiomer, or any combination or ratio of enantiomers (i.e., scalemic mixture). In addition, the small molecule drug may possess one or more geometric isomers. With respect to geometric isomers, a composition can comprise a single geometric isomer or a mixture of two or more geometric isomers. A small molecule drug as disclosed herein can be in its customary active form, or may possess some degree of modification. For example, a small molecule drug may have a targeting agent, tag, or transporter attached thereto, prior to or after covalent attachment of an oligomer. Alternatively, the small molecule drug may possess a lipophilic moiety attached thereto, such as a phospholipid (e.g., distearoylphosphatidylethanolamine or "DSPE," dipalmitoylphosphatidylethanolamine or "DPPE," and so forth) or a small fatty acid. In some instances, however, it is disclosed that the small molecule drug moiety does not include attachment to a lipophilic moiety.

Amino groups on calicimetics provide a point of attachment between the calcimimetic and the oligomer.

There are a number of examples of suitable oligomers activated with one or more functional groups that are useful for forming covalent linkages with available amines of a calcimimetics. Specific examples, along with the corresponding conjugate, are provided in Table 1, below. In the table, the variable (n) represents the number of repeating monomeric units and "-NH-(Cal)" represents the residue of the calcimimetic following conjugation to the oligomer. While each oligomeric portion [e.g., (OCH₂CH₂)ₙ or (CH₂CH₂O)ₙ] presented in Table 1 terminates in a "CH₃" group, other groups (such as H and benzyl) can be substituted therefor.

**Table 1**

| **Amine-Selective Oligomers Activated with a Funtional Group and the Calcimimetic Conjugate Formed Therefrom** | |
|---|---|
| Polymeric Reagent | Corresponding Conjugate |
| 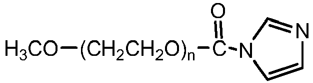 | 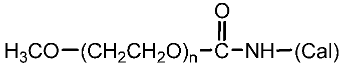 |
| mPEG-Oxycarbonylimidazole Reagent | Carbamate Linkage |
| 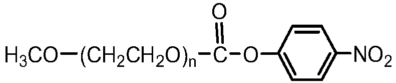 | 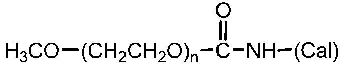 |
| mPEG Nitrophenyl Reagent | Carbamate Linkage |
| 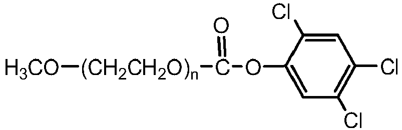 | 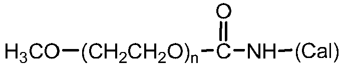 |
| mPEG-Trichlorophenyl Carbonate Reagent | Carbamate Linkage |
| 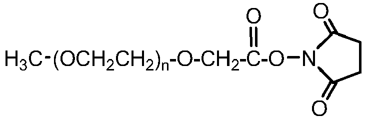 | 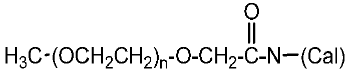 |
| mPEG-Succinimidyl Reagent | Amide Linkage |
| 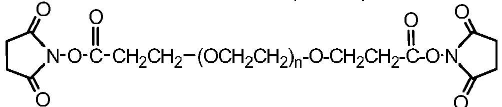 | 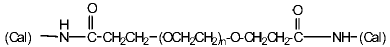 |
| Homobifunctional PEG-Succinimidyl Reagent | Amide Linkages |
| 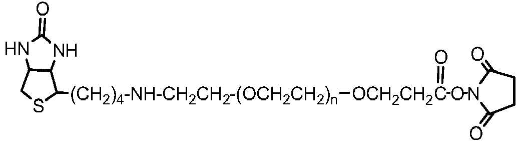 | 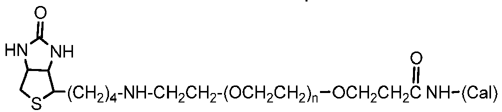 |
| Heterobifunctional PEG-Succinimidyl Reagent | Amide Linkage |

| Polymeric Reagent | Corresponding Conjugate |
|---|---|
| 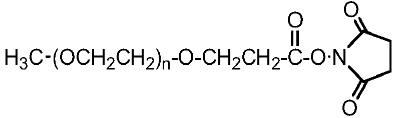 | 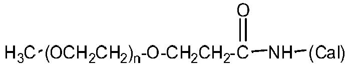 |
| mPEG-Succinimidyl Reagent | Amide Linkage |
| 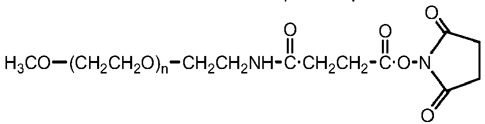 | 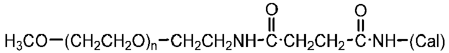 |
| mPEG-Succinimdyl Reagent | Amide Linkage |
| 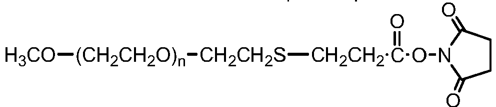 | 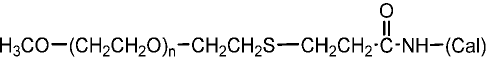 |
| mPEG Succinimidyl Reagent | Amide Linkage |
| 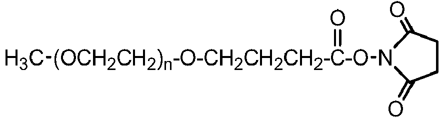 | 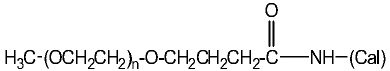 |
| mPEG-Succinimidyl Reagent | Amide Linkage |
| 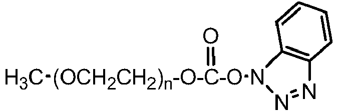 | 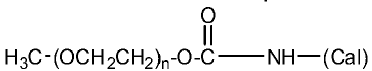 |
| mPEG-Benzotriazole Carbonate Reagent | Carbamate Linkage |
| 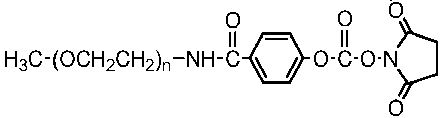 | 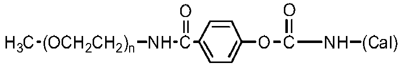 |
| mPEG-Succinimidyl Reagent | Carbamate Linkage |
| 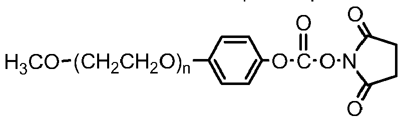 | 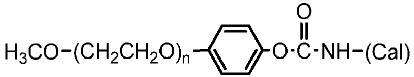 |
| mPEG-Succinimidyl Reagent | Amide Linkage |
| 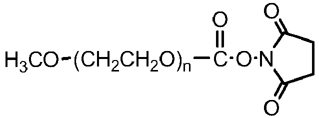 | 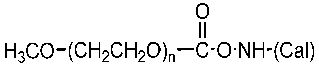 |
| mPEG Succinimidyl Carbonate Reagent | Carbamate Linkage |
| 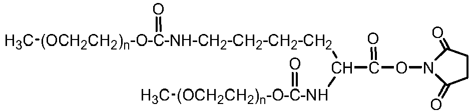 | 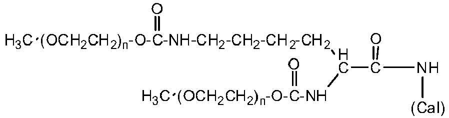 |
| Branched mPEG2-N-Hydroxysuccinimide Reagent | Amide Linkage |
| 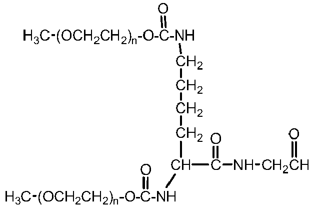 | 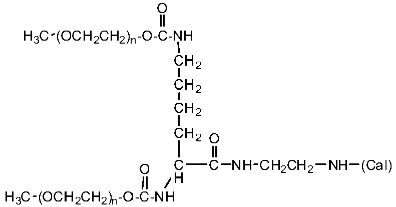 |
| Branched mPEG2-Aldehyde Reagent | Secondary Amine Linkage |
| 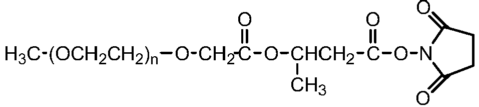 | 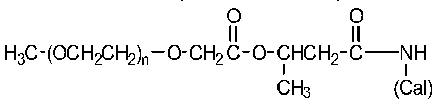 |
| mPEG-Succinimidyl Reagent | Amide Linkage |
| 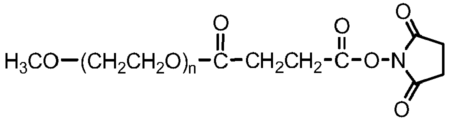 | 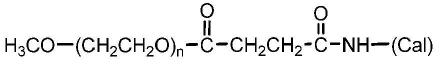 |
| mPEG-Succinimidyl Reagent | Amide Linkage |
| 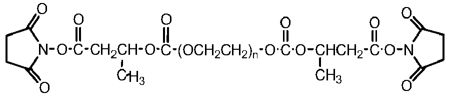 | 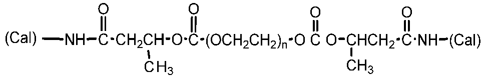 |
| Homobifunctional PEG-Succinimidyl Reagent | Amide Linkages |
| 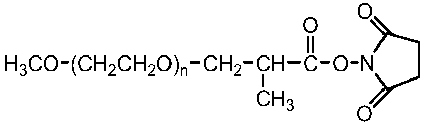 | 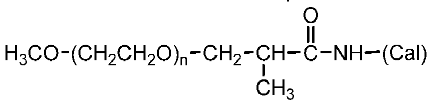 |
| mPEG-Succinimidyl Reagent | Amide Linkage |
| 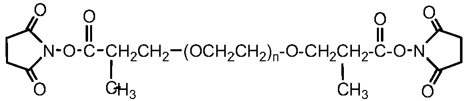 | 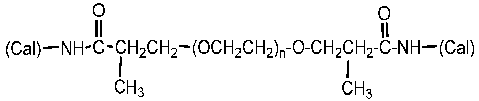 |
| Homobifunctional PEG-Succinimidyl Propionate Reagent | Amide Linkages |
| 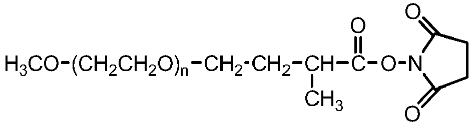 | 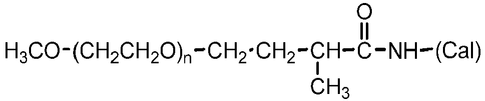 |
| mPEG-Succinimidyl Reagent | Amide Linkage |
| 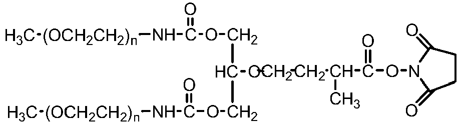 | 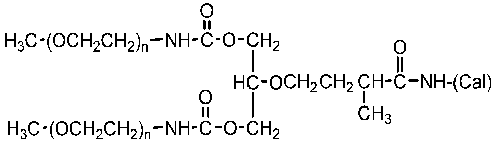 |
| Branched mPEG2-N-Hydroxysuccinimide Reagent | Amide Linkage |
| 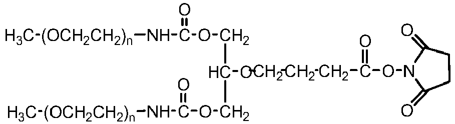 | 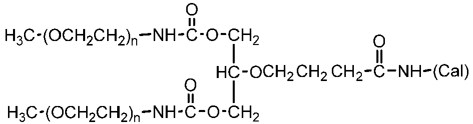 |
| Branched mPEG2-N-Hydroxysuccinimide Reagent | Amide Linkage |
| 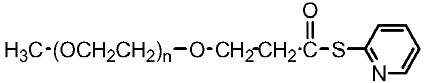 | 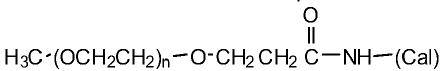 |
| mPEG-Thioester Reagent | Amide Linkage |
| 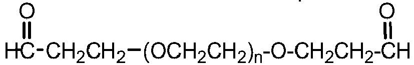 | 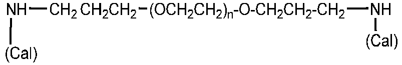 |
| Homobifunctional PEG Propionaldehyde Reagent | Secondary Amine Linkages |
| 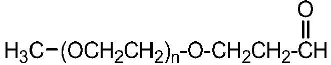 | H₃C-(OCH₂CH₂)ₙ-O-CH₂CH₂-CH₂₋NH-(Cal) |
| mPEG Propionaldehyde Reagent | Secondary Amine Linkage |
| 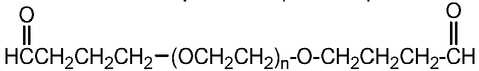 Homobifunctional PEG Butyraldehyde Reagent | 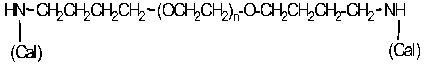 |
| | Secondary Amine Linkages |
| 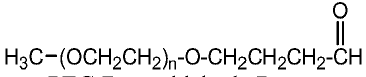 | 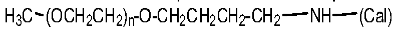 |
| mPEG Butryaldehyde Reagent | Secondary Amine Linkage |
| 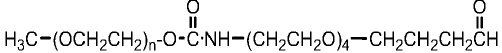 mPEG Butryaldehyde Reagent | 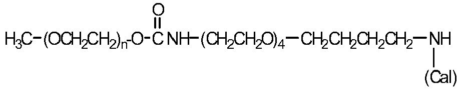 |
| | Secondary Amine Linkage |
| 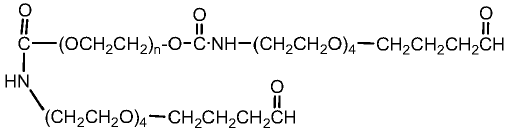 | 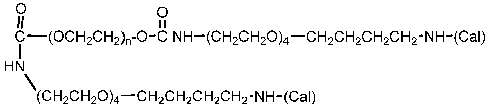 |
| Homobifunctional PEG Butryaldehyde Reagent | Secondary Amine Linkages |
| 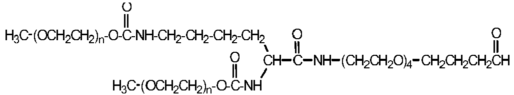 | 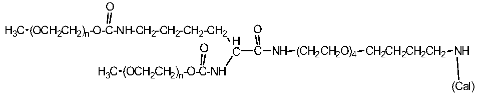 |
| Branched mPEG2 Butyraldehyde Reagent | Secondary Amine Linkage |
| 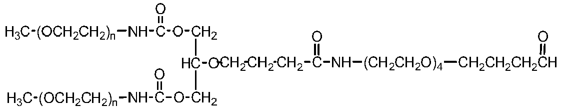 | 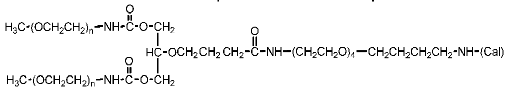 |
| Branched mPEG2 Butyraldehyde Reagent | Secondary Amine Linkage |
| 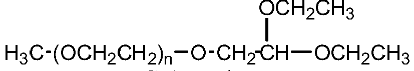 | H₃C-(OCH₂CH₂)ₙ-O-CH₂CH₂-NH-(Cal) |
| mPEG Acetal Reagent | Secondary Amine Linkage |
| 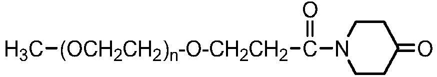 | 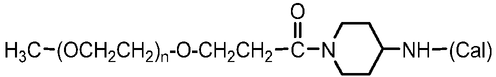 |
| mPEG Piperidone Reagent | Secondary Amine Linkage (to a secondary carbon) |
| 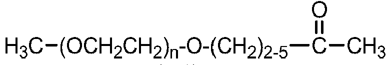 | 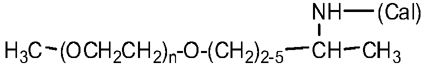 |
| mPEG Methylketone Reagent | secondary amine linkage (to a secondary carbon) |
| 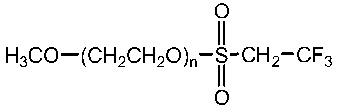 | H₃CO-(CH₂CH₂O)ₙ-CH₂CH₂-NH-(Cal) |
| mPEG tresylate Reagent | Secondary Amine Linkage |
| 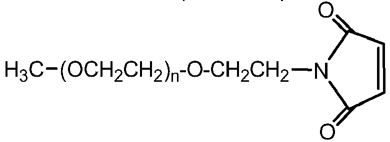 | 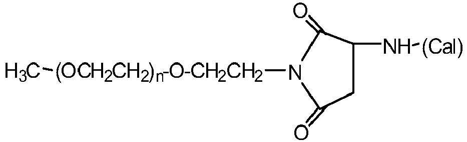 |
| mPEG Maleimide Reagent (under certain reaction conditions such as pH > 8) | Secondary Amine Linkage |
| 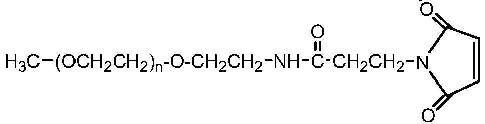 | 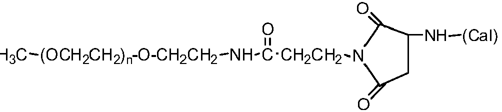 |
| mPEG Maleimide Reagent (under certain reaction conditions such as pH > 8) | Secondary Amine Linkage |
| 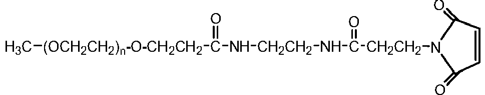 | 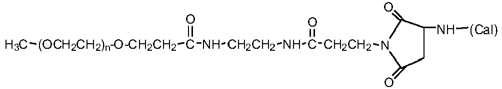 |
| mPEG Maleimide Reagent (under certain reaction conditions such as pH > 8) | Secondary Amine Linkage |
| 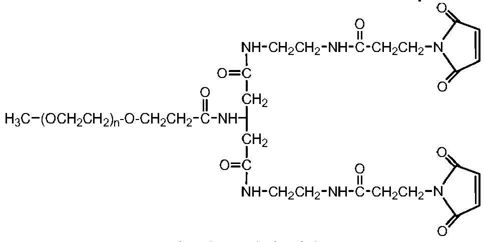 | 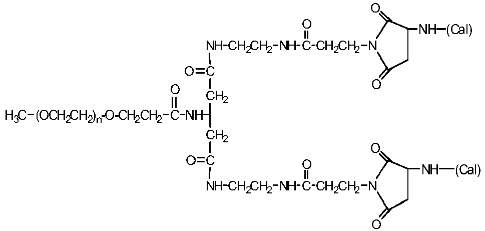 |
| mPEG Forked Maleimide Reagent (under certain reaction conditions such as pH > 8) | Secondary Amine Linkages |
| 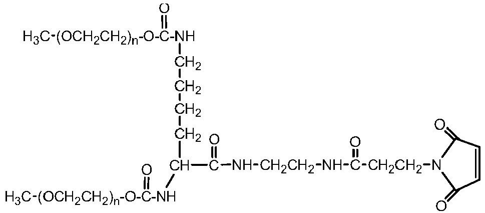 | 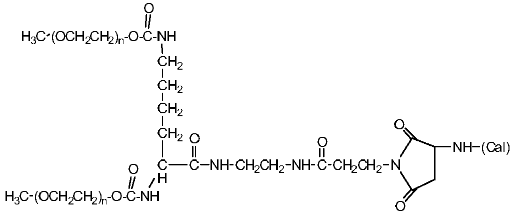 |
| branched mPEG2 Maleimide Reagent (under certain reaction conditions such as pH > 8) | Secondary Amine Linkage |
| 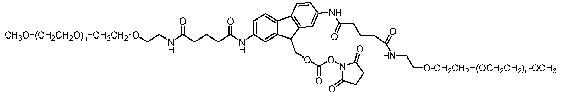 | 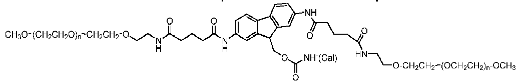 |
| | Releasable linkage |
| 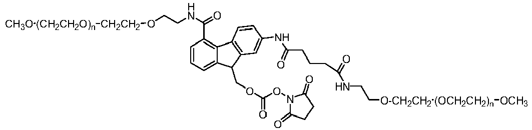 | 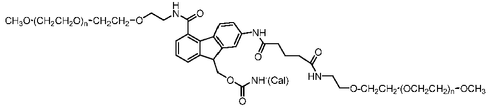 |
| | Releasable Linkage |
| 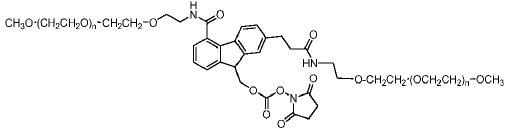 | 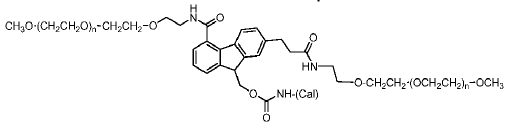 |
| | Releasable Linkage |
| 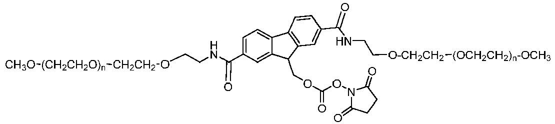 | 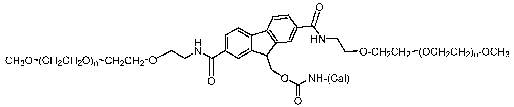 |
| | Releasable Linkage |

Conjugation of an oligomer to an amino group of a calcimimetic can be accomplished by a variety of techniques. In one approach, a calcimimetic can be conjugated to an oligomer functionalized with a succinimidyl derivative (or other activated ester or carbonate group, wherein approaches similar to those described for these alternative activated ester group-containing polymeric reagents can be used). In this approach, the oligomer bearing a succinimidyl derivative can be attached to the calcimimetic in an aqueous media at a pH of 7 to 9.0. In addition, an amide linkage can be formed by reacting an amine-terminated water-soluble, nonpeptidic oligomer with a calcimimetic bearing an activating a carboxylic acid group.

Accordingly, each oligomer is composed of up to three different monomer types selected from the group consisting of: alkylene oxide, such as ethylene oxide or propylene oxide; olefinic alcohol, such as vinyl alcohol, 1-propenol or 2-propenol; vinyl pyrrolidone; hydroxyalkyl methacrylamide or hydroxyalkyl methacrylate, where alkyl is preferably methyl; α-hydroxy acid, such as lactic acid or glycolic acid; phosphazene, oxazoline, amino acids, carbohydrates such as monosaccharides, saccharide or mannitol; and N-acryloylmorpholine. Preferred monomer types include alkylene oxide, olefinic alcohol, hydroxyalkyl methacrylamide or methacrylate, N-acryloylmorpholine, and α-hydroxy acid. Preferably, each oligomer is, independently, a co-oligomer of two monomer types selected from this group, or, more preferably, is a homo-oligomer of one monomer type selected from this group.

The two monomer types in a co-oligomer may be of the same monomer type, for example, two alkylene oxides, such as ethylene oxide and propylene oxide. Preferably, the oligomer is a homo-oligomer of ethylene oxide. Usually, although not necessarily, the terminus (or termini) of the oligomer that is not covalently attached to a small molecule is capped to render it unreactive. Alternatively, the terminus may include a reactive group. When the terminus is a reactive group, the reactive group is either selected such that it is unreactive under the conditions of formation of the final oligomer or during covalent attachment of the oligomer to a small molecule drug, or it is protected as necessary. One common end-functional group is hydroxyl or -OH, particularly for oligoethylene oxides.

The water-soluble, non-peptidic oligomer (e.g., "POLY" in various structures provided herein) can have any of a number of different geometries. For example, it can be linear, branched, or forked. Most typically, the water-soluble, non-peptidic oligomer is linear or is branched, for example, having one branch point.

With respect to the compounds of the invention, the molecular weight of the oligomer can either be relatively small or relatively large. For example, and without being bound by theory, it is believed that orally available calcimimetics (in unconjugated form) can substantially retain the ability to be orally available when a relatively small molecular weight (e.g., less than 2,000 Daltons) oligomer is present in the compound. In addition, and again without being bound by theory, even if inclusion of a relatively large (e.g., a molecular weight of from between 2,000 Daltons to about 80,000 Daltons) would require intravenous administration, such administration may not represent a significant drawback inasmuch as patients receiving calicimimetics may already be relying on dialysis, thereby having relatively easy access to the infrastructure and health care providers necessary to administer intravenous drugs.

When the molecular weight of the water-soluble, non-peptidic oligomer, excluding the linker portion, in the compound is relatively small (e.g., less than 2,000 Daltons), exemplary values of the molecular weight of the water-soluble oligomer include: below about 1500; below about 1450; below about 1400; below about 1350; below about 1300; below about 1250; below about 1200; below about 1150; below about 1100; below about 1050; below about 1000; below about 950; below about 900; below about 850; below about 800; below about 750; below about 700; below about 650; below about 600; below about 550; below about 500; below about 450; below about 400; below about 350; below about 300; below about 250; below about 200; and below about 100 Daltons.

Exemplary ranges of molecular weights of the water-soluble, non-peptidic oligomer (excluding the linker) include: from about 100 to about 1400 Daltons; from about 100 to about 1200 Daltons; from about 100 to about 800 Daltons; from about 100 to about 500 Daltons; from about 100 to about 400 Daltons; from about 200 to about 500 Daltons; from about 200 to about 400 Daltons; from about 75 to 1000 Daltons; and from about 75 to about 750 Daltons.

Preferably, the number of monomers in the water-soluble, non-peptidic oligomer falls within one or more of the following ranges: between about 1 and about 30 (inclusive); between about 1 and about 25; between about 1 and about 20; between about 1 and about 15; between about 1 and about 12; between about 1 and about 10. In certain instances, the number of monomers in series in the oligomer (and the corresponding conjugate) is one of 1, 2, 3, 4, 5, 6, 7, or 8. In additional embodiments, the oligomer (and the corresponding conjugate) contains 9, 10, 11, 12, 13, 14, 15, 16, 17, 18, 19, or 20 monomers. In yet further embodiments, the oligomer (and the corresponding conjugate) possesses 21, 22, 23, 24, 25, 26, 27, 28, 29 or 30 monomers in series. Thus, for example, when the water-soluble and non-peptidic polymer includes CH₃-(OCH₂CH₂)ₙ-, "n" is an integer that can be 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30, and can fall within one or more of the following ranges: between about 1 and about 25; between about 1 and about 20; between about 1 and about 15; between about 1 and about 12; between about 1 and about 10.

When the water-soluble, non-peptidic oligomer has 1, 2, 3, 4, 5, 6, 7, 8, 9, or 10 monomers, these values correspond to a methoxy end-capped oligo(ethylene oxide) having a molecular weights of about 75, 119, 163, 207, 251, 295, 339, 383, 427, and 471 Daltons, respectively. When the oligomer has 11, 12, 13, 14, or 15 monomers, these values correspond to methoxy end-capped oligo(ethylene oxide) having molecular weights corresponding to about 515, 559, 603, 647, and 691 Daltons, respectively.

When the molecular weight of the water-soluble, non-peptidic oligomer, excluding the linker portion, in the compound is relatively large (e.g., greater than 2,000 Daltons), the weight can fall within the range of 2,000 Daltons to about 150,000 Daltons. Exemplary ranges, however, include weight-average molecular weights in the range of from about 3,000 Daltons to about 120,000 Daltons; in the range of from about 5,000 Daltons to about 110,000 Daltons; in the range of from greater than 5,000 Daltons to about 100,000 Daltons, in the range of from about 6,000 Daltons to about 90,000 Daltons, in the range of from about 10,000 Daltons to about 85,000 Daltons, in the range of greater than 10,000 Daltons to about 85,000 Daltons, in the range of from about 20,000 Daltons to about 85,000 Daltons, in the range of from about 53,000 Daltons to about 85,000 Daltons, in the range of from about 25,000 Daltons to about 120,000 Daltons, in the range of from about 29,000 Daltons to about 120,000 Daltons, in the range of from about 35,000 Daltons to about 120,000 Daltons, and in the range of from about 40,000 Daltons to about 120,000 Daltons. For any given oligomer, poly(ethylene oxides) having a molecular weight in one or more of these ranges are preferred.

Exemplary weight-average molecular weights for the water-soluble oligomer include about 2,200 Daltons, about 2,500 Daltons, about 3,000 Daltons, about 4,000 Daltons, about 4,400 Daltons, about 4,500 Daltons, about 5,000 Daltons, about 5,500 Daltons, about 6,000 Daltons, about 7,000 Daltons, about 7,500 Daltons, about 8,000 Daltons, about 9,000 Daltons, about 10,000 Daltons, about 11,000 Daltons, about 12,000 Daltons, about 13,000 Daltons, about 14,000 Daltons, about 15,000 Daltons, about 20,000 Daltons, about 22,500 Daltons, about 25,000 Daltons, about 30,000 Daltons, about 35,000 Daltons, about 40,000 Daltons, about 45,000 Daltons, about 50,000 Daltons, about 55,000 Daltons, about 60,000 Daltons, about 65,000 Daltons, about 70,000 Daltons, and about 75,000 Daltons. Branched versions of the water-soluble oligomer (e.g., a branched 40,000 Dalton water-soluble oligomer comprised of two 20,000 Dalton oligomers) having a total molecular weight of any of the foregoing can also be used. In one or more embodiments, the conjugate will not have any PEG moieties attached, either directly or indirectly, with a PEG having a weight average molecular weight of less than about 6,000 Daltons.

When used as the polymer, PEGs will typically comprise a number of (OCH₂CH₂) monomers [or (CH₂CH₂O) monomers, depending on how the PEG is defined]. As used throughout the description, the number of repeating units is identified by the subscript "*n*" in "(OCH₂CH₂)ₙ." Thus, the value of (n) typically falls within one or more of the following ranges: from 2 to about 3400, from about 100 to about 2300, from about 100 to about 2270, from about 136 to about 2050, from about 225 to about 1930, from about 450 to about 1930, from about 1200 to about 1930, from about 568 to about 2727, from about 660 to about 2730, from about 795 to about 2730, from about 795 to about 2730, from about 909 to about 2730, and from about 1,200 to about 1,900. For any given polymer in which the molecular weight is known, it is possible to determine the number of repeating units (i.e., "n") by dividing the total weight-average molecular weight of the polymer by the molecular weight of the repeating monomer

When the water-soluble, non-peptidic oligomer is attached to the calcimimetic (in contrast to the step-wise addition of one or more monomers to effectively "grow" the oligomer onto the calcimimetic), it is preferred that the composition containing an activated form of the water-soluble, non-peptidic oligomer be monodisperse. In those instances, however, where a bimodal composition is employed, the composition will possess a bimodal distribution centering around any two of the above numbers of monomers. For instance, a bimodal oligomer may have any one of the following exemplary combinations of monomer subunits: 1-2, 1-3, 1-4, 1-5, 1-6, 1-7, 1-8, 1-9, 1-10, and so forth; 2-3, 2-4, 2-5, 2-6, 2-7, 2-8, 2-9, 2-10, and so forth; 3-4, 3-5, 3-6, 3-7, 3-8, 3-9, 3-10, and so forth; 4-5, 4-6, 4-7, 4-8, 4-9, 4-10, and so forth; 5-6, 5-7, 5-8, 5-9, 5-10, and so forth; 6-7, 6-8, 6-9, 6-10, and so forth; 7-8, 7-9, 7-10, and so forth; and 8-9, 8-10, and so forth.

In some instances, the composition containing an activated form of the water-soluble, non-peptidic oligomer will be trimodal or even tetramodal, possessing a range of monomers units as previously described. Oligomer compositions possessing a well-defined mixture of oligomers (i.e., being bimodal, trimodal, tetramodal, and so forth) can be prepared by mixing purified monodisperse oligomers to obtain a desired profile of oligomers (a mixture of two oligomers differing only in the number of monomers is bimodal; a mixture of three oligomers differing only in the number of monomers is trimodal; a mixture of four oligomers differing only in the number of monomers is tetramodal), or alternatively, can be obtained from column chromatography of a polydisperse oligomer by recovering the "center cut", to obtain a mixture of oligomers in a desired and defined molecular weight range.

It is preferred that the water-soluble, non-peptidic oligomer is obtained from a composition that is unimolecular or monodisperse. That is, the oligomers in the composition possess the same discrete molecular weight value rather than a distribution of molecular weights. Some monodisperse oligomers can be purchased from commercial sources such as those available from Sigma-Aldrich, or alternatively, can be prepared directly from commercially available starting materials such as Sigma-Aldrich. Water-soluble, non-peptidic oligomers can be prepared as described in Chen Y., Baker, G.L., J. Org. Chem., 6870-6873 (1999), WO 02/098949, and U.S. Patent Application Publication No. 2005/0136031.

When present, the spacer moiety, also referred herein as a "linker," (through which the water-soluble and non-peptidic polymer is attached to the calcimimetic) may be a single bond, a single atom, such as an oxygen atom or a sulfur atom, two atoms, or a number of atoms. A spacer moiety is typically but is not necessarily linear in nature. The spacer moiety, "X," can be stable or releasable. With respect to enzymes found within living systems, the spacer moiety, "X," can be stable to such enzymes or releasable upon interaction with such enzymes. Preferably, the spacer moiety "X" is one having a chain length of less than about 12 atoms, and preferably less than about 10 atoms, and even more preferably less than about 8 atoms and even more preferably less than about 5 atoms, whereby length is meant the number of atoms in a single chain, not counting substituents. For instance, a urea linkage such as this, R_{oligomer}-NH-(C=O)-NH-R'_{drug}, is considered to have a chain length of 3 atoms (-NH-C(O)-NH-). In selected embodiments, the linkage does not comprise further spacer groups.

In some instances, the spacer moiety "X" (or "linkage") comprises an ether, amide, urethane, amine, thioether, urea, or a carbon-carbon bond. Functional groups such as those discussed below, and illustrated in the examples, are typically used for forming the linkages. The spacer moiety may less preferably also comprise (or be adjacent to or flanked by) other atoms, as described further below.

More specifically, in selected embodiments, a spacer moiety (or "linkage") of the invention, X, may be any of the following: "-" (i.e., a covalent bond, that may be stable or degradable, between the calcimimetic residue and the water-soluble, non-peptidic oligomer), -C(O)O-, -OC(O)-, -CH₂-C(O)O-, -CH₂-OC(O)-, -C(O)O-CH₂-, -OC(O)-CH₂-, -O-, -NH-, -S-, -C(O)-, C(O)-NH, NH-C(O)-NH, O-C(O)-NH, -C(S)-, -CH₂-, -CH₂-CH₂-, -CH₂-CH₂-CH₂-, -CH₂-CH₂-CH₂-CH₂-, -O-CH₂-, -CH₂-O-, -O-CH₂-CH₂-, -CH₂-O-CH₂-, -CH₂-CH₂-O-, -O-CH₂-CH₂-CH₂-, -CH₂-O-CH₂-CH₂-, -CH₂-CH₂-O-CH₂-, -CH₂-CH₂-CH₂-O-, -O-CH₂-CH₂-CH₂-CH₂-, -CH₂-O-CH₂-CH₂-CH₂-, -CH₂-CH₂-O-CH₂-CH₂-, -CH₂-CH₂-CH₂-O-CH₂-, -CH₂-CH₂-CH₂-CH₂-O-, -C(O)-NH-CH₂-, -C(O)-NH-CH₂-CH₂-, -CH₂-C(O)-NH-CH₂-, -CH₂-CH₂-C(O)-NH-, -C(O)-NH-CH₂-CH₂-CH₂-, -CH₂-C(O)-NH-CH₂-CH₂-, -CH₂-CH₂-C(O)-NH-CH₂-, -CH₂-CH₂-CH₂-C(O)-NH-, -C(O)-NH-CH₂-CH₂-CH₂-CH₂-, -CH₂-C(O)-NH-CH₂-CH₂-CH₂-, -CH₂-CH₂-C(O)-NH-CH₂-CH₂-, -CH₂-CH₂-CH₂-C(O)-NH-CH₂-, -CH₂-CH₂-CH₂-C(O)-NH-CH₂-CH₂-, -CH₂-CH₂-CH₂-CH₂-C(O)-NH-, -NH-C(O)-CH₂-, -CH₂-NH-C(O)-CH₂-, -CH₂-CH₂-NH-C(O)-CH₂-, -NH-C(O)-CH₂-CH₂-, -CH₂-NH-C(O)-CH₂-CH₂, -CH₂-CH₂-NH-C(O)-CH₂-CH₂, -C(O)-NH-CH₂-, -C(O)-NH-CH₂-CH₂-, -O-C(O)-NH-CH₂-, -O-C(O)-NH-CH₂-CH₂-, -NH-CH₂-, -NH-CH₂-CH₂-, -CH₂-NH-CH₂-, -CH₂-CH₂-NH-CH₂-, -C(O)-CH₂-, -C(O)-CH₂-CH₂-, -CH₂-C(O)-CH₂-, -CH₂-CH₂-C(O)-CH₂-, -CH₂-CH₂-C(O)-CH₂-CH₂-, -CH₂-CH₂-C(O)-, -CH₂-CH₂-CH₂-C(O)-NH-CH₂-CH₂-NH-, -CH₂-CH₂-CH₂-C(O)-NH-CH₂-CH₂-NH-C(O)-, -CH₂-CH₂-CH₂-C(O)-NH-CH₂-CH₂-NH-C(O)-CH₂-, bivalent cycloalkyl group, -N(R⁶)-, R⁶ is H or an organic radical selected from the group consisting of alkyl, substituted alkyl, alkenyl, substituted alkenyl, alkynyl, substituted alkynyl, aryl and substituted aryl.

For purposes of the present invention, however, a group of atoms is not considered a linkage when it is immediately adjacent to an oligomer segment, and the group of atoms is the same as a monomer of the oligomer such that the group would represent a mere extension of the oligomer chain.

The linkage "X" between the water-soluble, non-peptidic oligomer and the small molecule is typically formed by reaction of a functional group on a terminus of the oligomer (or nascent oligomer when it is desired to "grow" the oligomer onto the calcimimetic) with a corresponding functional group within the calcimimetic. Illustrative reactions are described briefly below. For example, an amino group on an oligomer may be reacted with a carboxylic acid or an activated carboxylic acid derivative on the small molecule, or vice versa, to produce an amide linkage. Alternatively, reaction of an amine on an oligomer with an activated carbonate (e.g., succinimidyl or benzotriazyl carbonate) on the drug, or vice versa, forms a carbamate linkage. Reaction of an amine on an oligomer with an isocyanate (R-N=C=O) on a drug, or vice versa, forms a urea linkage (R-NH-(C=O)-NH-R'). Further, reaction of an alcohol (alkoxide) group on an oligomer with an alkyl halide, or halide group within a drug, or vice versa, forms an ether linkage. In yet another coupling approach, a small molecule having an aldehyde function is coupled to an oligomer amino group by reductive amination, resulting in formation of a secondary amine linkage between the oligomer and the small molecule.

A particularly preferred water-soluble, non-peptidic oligomer is an oligomer bearing an aldehyde functional group. In this regard, the oligomer will have the following structure: CH₃O-(CH₂-CH₂-O)ₙ-(CH₂)ₚ-C(O)H, wherein (n) is one of 1, 2, 3, 4, 5, 6, 7, 8, 9 and 10 and (p) is one of 1, 2, 3, 4, 5, 6 and 7. Preferred (n) values include 3, 5 and 7 and preferred (p) values 2, 3 and 4. In addition, the carbon atom alpha to the -C(O)H moiety can optionally be substituted with alkyl.

Typically, all but one termini of the water-soluble, non-peptidic oligomer not bearing a functional group is capped to render it unreactive. When the oligomer includes a further functional group at a terminus other than that intended for formation of a conjugate, that group is either selected such that it is unreactive under the conditions of formation of the linkage "X," or it is protected during the formation of the linkage "X."

As stated above, the water-soluble, non-peptidic oligomer includes at least one functional group prior to conjugation. The functional group typically comprises an electrophilic or nucleophilic group for covalent attachment to a small molecule, depending upon the reactive group contained within or introduced into the small molecule. Examples of nucleophilic groups that may be present in either the oligomer or the small molecule include hydroxyl, amine, hydrazine (-NHNH₂), hydrazide (-C(O)NHNH₂), and thiol. Preferred nucleophiles include amine, hydrazine, hydrazide, and thiol, particularly amine. Most small molecule drugs for covalent attachment to an oligomer will possess a free hydroxyl, amino, thio, aldehyde, ketone, or carboxyl group.

Examples of electrophilic functional groups that may be present in either the oligomer or the small molecule include carboxylic acid, carboxylic ester, particularly imide esters, orthoester, carbonate, isocyanate, isothiocyanate, aldehyde, ketone, thione, alkenyl, acrylate, methacrylate, acrylamide, sulfone, maleimide, disulfide, iodo, epoxy, sulfonate, thiosulfonate, silane, alkoxysilane, and halosilane. More specific examples of these groups include succinimidyl ester or carbonate, imidazoyl ester or carbonate, benzotriazole ester or carbonate, vinyl sulfone, chloroethylsulfone, vinylpyridine, pyridyl disulfide, iodoacetamide, glyoxal, dione, mesylate, tosylate, and tresylate (2,2,2-trifluoroethanesulfonate).

Also included are sulfur analogs of several of these groups, such as thione, thione hydrate, thioketal, is 2-thiazolidine thione, etc., as well as hydrates or protected derivatives of any of the above moieties (e.g. aldehyde hydrate, hemiacetal, acetal, ketone hydrate, hemiketal, ketal, thioketal, thioacetal).

An "activated derivative" of a carboxylic acid refers to a carboxylic acid derivative that reacts readily with nucleophiles, generally much more readily than the underivatized carboxylic acid. Activated carboxylic acids include, for example, acid halides (such as acid chlorides), anhydrides, carbonates, and esters. Such esters include imide esters, of the general form -(CO)O-N[(CO)-]₂; for example, N-hydroxysuccinimidyl (NHS) esters or N-hydroxyphthalimidyl esters. Also preferred are imidazolyl esters and benzotriazole esters. Particularly preferred are activated propionic acid or butanoic acid esters, as described in co-owned U.S. Patent No. 5,672,662. These include groups of the form -(CH₂)₂₋₃C(=O)O-Q, where Q is preferably selected from N-succinimide, N-sulfosuccinimide, N-phthalimide, N-glutarimide, N-tetrahydrophthalimide, N-norbornene-2,3-dicarboximide, benzotriazole, 7-azabenzotriazole, and imidazole.

Other preferred electrophilic groups include succinimidyl carbonate, maleimide, benzotriazole carbonate, glycidyl ether, imidazoyl carbonate, p-nitrophenyl carbonate, acrylate, tresylate, aldehyde, and orthopyridyl disulfide.

These electrophilic groups are subject to reaction with nucleophiles, e.g., hydroxy, thio, or amino groups, to produce various bond types. Carboxylic acids and activated derivatives thereof, which include orthoesters, succinimidyl esters, imidazolyl esters, and benzotriazole esters, react with the above types of nucleophiles to form esters, thioesters, and amides, respectively, of which amides are the most hydrolytically stable. Carbonates, including succinimidyl, imidazolyl, and benzotriazole carbonates, react with amino groups to form carbamates. Isocyanates (R-N=C=O) react with hydroxyl or amino groups to form, respectively, carbamate (RNH-C(O)-OR') or urea (RNH-C(O)-NHR') linkages. Aldehydes, ketones, glyoxals, diones and their hydrates or alcohol adducts (i.e., aldehyde hydrate, hemiacetal, acetal, ketone hydrate, hemiketal, and ketal) are preferably reacted with amines, followed by reduction of the resulting imine, if desired, to provide an amine linkage (reductive amination).

Several of the electrophilic functional groups include electrophilic double bonds to which nucleophilic groups, such as thiols, can be added, to form, for example, thioether bonds. These groups include maleimides, vinyl sulfones, vinyl pyridine, acrylates, methacrylates, and acrylamides. Other groups comprise leaving groups that can be displaced by a nucleophile; these include chloroethyl sulfone, pyridyl disulfides (which include a cleavable S-S bond), iodoacetamide, mesylate, tosylate, thiosulfonate, and tresylate. Epoxides react by ring opening by a nucleophile, to form, for example, an ether or amine bond. Reactions involving complementary reactive groups such as those noted above on the oligomer and the small molecule are utilized to prepare the conjugates of the invention.

It is disclosed that the calcimimetic may not have a functional group suited for conjugation. In this instance, it is possible to modify (or "functionalize") the "original" calcimimetic so that it does have a functional group suited for conjugation. For example, if the calcimimetic has an amide group, but an amine group is desired, it is possible to modify the amide group to an amine group by way of a Hofmann rearrangement, Curtius rearrangement (once the amide is converted to an azide) or Lossen rearrangement (once amide is concerted to hydroxamide followed by treatment with tolyene-2-sulfonyl chloride/base).

It is possible to prepare a conjugate of small molecule calcimimetic bearing a carboxyl group wherein the carboxyl group-bearing small molecule calcimimetic is coupled to an amino-terminated oligomeric ethylene glycol, to provide a conjugate having an amide group covalently linking the small molecule calcimimetic to the oligomer. This can be performed, for example, by combining the carboxyl group-bearing small molecule calcimimetic with the amino-terminated oligomeric ethylene glycol in the presence of a coupling reagent, (such as dicyclohexylcarbodiimide or "DCC") in an anhydrous organic solvent.

Further, it is possible to prepare a conjugate of a small molecule calcimimetic bearing a hydroxyl group wherein the hydroxyl group-bearing small molecule calcimimetic is coupled to an oligomeric ethylene glycol halide to result in an ether (-O-) linked small molecule conjugate. This can be performed, for example, by using sodium hydride to deprotonate the hydroxyl group followed by reaction with a halide-terminated oligomeric ethylene glycol.

Further, it is possible to prepare a conjugate of a small molecule calcimimetic bearing a hydroxyl group (such as, for example, the calcimimetic having structures encompassed within at least one of Formulae I and II) wherein the hydroxyl group-bearing small molecule calcimimetic is coupled to an oligomeric ethylene glycol bearing an haloformate group [e.g., CH₃(OCH₂CH₂OC(O)-halo, where halo is chloro, bromo, iodo] to result in an carbonate [-O-C(O)-O-] linked small molecule conjugate. This can be performed, for example, by combining a calcimimetic and an oligomeric ethylene glycol bearing a haloformate group in the presence of a nucleophilic catalyst (such as 4-dimethylaminopyridine or "DMAP") to thereby result in the corresponding carbonate-linked conjugate.

It is disclosed that in another example, it is possible to prepare a conjugate of a small molecule calcimimetic bearing a ketone group by first reducing the ketone group to form the corresponding hydroxyl group. Thereafter, the small molecule calcimimetic now bearing a hydroxyl group can be coupled as described herein.

In still another instance, it is possible to prepare a conjugate of a small molecule calcimimetic bearing an amine group. In one approach, the amine group-bearing small molecule calcimimetic and an aldehyde-bearing oligomer are dissolved in a suitable buffer after which a suitable reducing agent (e.g., NaCNBH₃) is added. Following reduction, the result is an amine linkage formed between the amine group of the amine group-containing small molecule calcimimetic and the carbonyl carbon of the aldehyde-bearing oligomer.

In another approach for preparing a conjugate of a small molecule calcimimetic bearing an amine group, a carboxylic acid-bearing oligomer and the amine group-bearing small molecule calcimimetic are combined, typically in the presence of a coupling reagent (e.g., DCC). The result is an amide linkage formed between the amine group of the amine group-containing small molecule calcimimetic and the carbonyl of the carboxylic acid-bearing oligomer.

The compounds of the invention may be administered *per se* or in the form of a pharmaceutically acceptable salt, and any reference to the compounds of the invention herein is intended to include pharmaceutically acceptable salts. If used, a salt of a compound as described herein should be both pharmacologically and pharmaceutically acceptable, but non-pharmaceutically acceptable salts may conveniently be used to prepare the free active compound or pharmaceutically acceptable salts thereof and are not excluded from the scope of this invention. Such pharmacologically and pharmaceutically acceptable salts can be prepared by reaction of the compound with an organic or inorganic acid, using standard methods detailed in the literature. Examples of useful salts include, those prepared from the following acids: hydrochloric, hydrobromic, sulfuric, nitric, phosphoric, maleic, acetic, salicyclic, p-toluenesulfonic, tartaric, citric, methanesulfonic, formic, malonic, succinic, naphthalene-2-sulphonic and benzenesulphonic. Also, pharmaceutically acceptable salts can be prepared as alkaline metal or alkaline earth salts, such as sodium, potassium, or calcium salts of a carboxylic acid group.

While it is believed that the full scope of the conjugates disclosed herein behave as described, an optimally sized oligomer can be identified as by testing a series of compounds, each having a difference oligomer size and the compound having the best activity is identified as a preferred compound.

With respect to bioavailability, one of ordinary skill in the art, using routine experimentation, can determine a best suited molecular size and linkage for improving oral bioavailability by first preparing a series of oligomers with different weights and functional groups and then obtaining the necessary clearance profiles by administering the conjugates to a patient and taking periodic blood and/or urine sampling. Once a series of clearance profiles have been obtained for each tested conjugate, a suitable conjugate can be identified.

Animal models (rodents and dogs) can also be used to study oral drug transport. In addition, non-*in vivo* methods include rodent everted gut excised tissue and Caco-2 cell monolayer tissue-culture models. These models are useful in predicting oral drug bioavailability.

The present invention also includes pharmaceutical preparations comprising a conjugate of the invention in combination with a pharmaceutical excipient. Generally, the conjugate itself will be in a solid form (e.g., a precipitate), which can be combined with a suitable pharmaceutical excipient that can be in either solid or liquid form.

Exemplary excipients include, those selected from the group consisting of carbohydrates, inorganic salts, antimicrobial agents, antioxidants, surfactants, buffers, acids, bases, and combinations thereof.

A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose and sorbose; disaccharides, such as lactose, sucrose, trehalose and cellobiose; polysaccharides, such as raffinose, melezitose, maltodextrins, dextrans and starches; and alditols, such as mannitol, xylitol, maltitol, lactitol, xylitol, sorbitol (glucitol), pyranosyl sorbitol and myoinositol.

The excipient can also include an inorganic salt or buffer such as citric acid, sodium chloride, potassium chloride, sodium sulfate, potassium nitrate, sodium phosphate monobasic, sodium phosphate dibasic, and combinations thereof.

The preparation may also include an antimicrobial agent for preventing or deterring microbial growth. Examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the preparation as well. Antioxidants are used to prevent oxidation, thereby preventing the deterioration of the conjugate or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant may be present as an excipient. Exemplary surfactants include: polysorbates, such as "Tween 20" and "Tween 80," and pluronics such as F68 and F88 (both of which are available from BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; and chelating agents, such as EDTA, zinc and other such suitable cations.

Pharmaceutically acceptable acids or bases may be present as an excipient in the preparation. Examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of the conjugate in the composition will vary depending on a number of factors, but will optimally be a therapeutically effective dose when the composition is stored in a unit dose container. A therapeutically effective dose can be determined experimentally by repeated administration of increasing amounts of the conjugate in order to determine which amount produces a clinically desired endpoint.

The amount of any individual excipient in the composition will vary depending on the activity of the excipient and particular needs of the composition. Typically, the optimal amount of any individual excipient is determined through routine experimentation, i.e., by preparing compositions containing varying amounts of the excipient (ranging from low to high), examining the stability and other parameters, and then determining the range at which optimal performance is attained with no significant adverse effects.

Generally, however, excipients will be present in the composition in an amount of about 1% to about 99% by weight, preferably from about 5%-98% by weight, more preferably from about 15-95% by weight of the excipient, with concentrations less than 30% by weight most preferred.

These foregoing pharmaceutical excipients along with other excipients and general teachings regarding pharmaceutical compositions are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D.C., 2000.

The pharmaceutical compositions can take any number of forms. Exemplary preparations are most preferably in a form suitable for oral administration such as a tablet, caplet, capsule, gel cap, troche, dispersion, suspension, solution, elixir, syrup, lozenge, transdermal patch, spray, suppository, and powder.

Oral dosage forms are preferred for those conjugates that are orally active, and include tablets, caplets, capsules, gel caps, suspensions, solutions, elixirs, and syrups, and can also comprise a plurality of granules, beads, powders or pellets that are optionally encapsulated. Such dosage forms are prepared using conventional methods known to those in the field of pharmaceutical formulation and described in the pertinent texts.

Tablets and caplets, for example, can be manufactured using standard tablet processing procedures and equipment. Direct compression and granulation techniques are preferred when preparing tablets or caplets containing the conjugates described herein. In addition to the conjugate, the tablets and caplets will generally contain inactive, pharmaceutically acceptable carrier materials such as binders, lubricants, disintegrants, fillers, stabilizers, surfactants, coloring agents and flow agents. Binders are used to impart cohesive qualities to a tablet, and thus ensure that the tablet remains intact. Suitable binder materials include, starch (including corn starch and pregelatinized starch), gelatin, sugars (including sucrose, glucose, dextrose and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, microcrystalline cellulose, ethyl cellulose and hydroxyethyl cellulose), and Veegum. Lubricants are used to facilitate tablet manufacture, promoting powder flow and preventing particle capping (i.e., particle breakage) when pressure is relieved. Useful lubricants are magnesium stearate, calcium stearate, and stearic acid. Disintegrants are used to facilitate disintegration of the tablet, and are generally starches, clays, celluloses, algins, gums, or crosslinked polymers. Fillers include, for example, materials such as silicon dioxide, titanium dioxide, alumina, talc, kaolin, powdered cellulose, and microcrystalline cellulose, as well as soluble materials such as mannitol, urea, sucrose, lactose, dextrose, sodium chloride, and sorbitol. Stabilizers, as well known in the art, are used to inhibit or retard drug decomposition reactions that include, by way of example, oxidative reactions.

Capsules are also preferred oral dosage forms, in which case the conjugate-containing composition can be encapsulated in the form of a liquid or gel (e.g., in the case of a gel cap) or solid (including particulates such as granules, beads, powders or pellets). Suitable capsules include hard and soft capsules, and are generally made of gelatin, starch, or a cellulosic material. Two-piece hard gelatin capsules are preferably sealed, such as with gelatin bands.

Included are parenteral formulations in the substantially dry form (typically as a lyophilizate or precipitate, which can be in the form of a powder or cake), as well as formulations prepared for injection, which are typically liquid and requires the step of reconstituting the dry form of parenteral formulation. Examples of suitable diluents for reconstituting solid compositions prior to injection include bacteriostatic water for injection, dextrose 5% in water, phosphate-buffered saline, Ringer's solution, saline, sterile water, deionized water, and combinations thereof.

In some cases, compositions intended for parenteral administration can take the form of nonaqueous solutions, suspensions, or emulsions, each typically being sterile. Examples of nonaqueous solvents or vehicles are propylene glycol, polyethylene glycol, vegetable oils, such as olive oil and corn oil, gelatin, and injectable organic esters such as ethyl oleate.

The parenteral formulations described herein can also contain adjuvants such as preserving, wetting, emulsifying, and dispersing agents. The formulations are rendered sterile by incorporation of a sterilizing agent, filtration through a bacteria-retaining filter, irradiation, or heat.

The conjugate can also be administered through the skin using conventional transdermal patch or other transdermal delivery system, wherein the conjugate is contained within a laminated structure that serves as a drug delivery device to be affixed to the skin. In such a structure, the conjugate is contained in a layer, or "reservoir," underlying an upper backing layer. The laminated structure can contain a single reservoir, or it can contain multiple reservoirs.

The conjugate can also be formulated into a suppository for rectal administration. With respect to suppositories, the conjugate is mixed with a suppository base material which is (e.g., an excipient that remains solid at room temperature but softens, melts or dissolves at body temperature) such as cocoa butter (theobroma oil), polyethylene glycols, glycerinated gelatin, fatty acids, and combinations thereof. Suppositories can be prepared by, for example, performing the following steps (not necessarily in the order presented): melting the suppository base material to form a melt; incorporating the conjugate (either before or after melting of the suppository base material); pouring the melt into a mold; cooling the melt (e.g., placing the melt-containing mold in a room temperature environment) to thereby form suppositories; and removing the suppositories from the mold.

There is described a method for administering a conjugate as provided herein to a patient suffering from a condition that is responsive to treatment with the conjugate. The method comprises administering, generally orally, a therapeutically effective amount of the conjugate (preferably provided as part of a pharmaceutical preparation). Other modes of administration are also contemplated, such as pulmonary, nasal, buccal, rectal, sublingual, transdermal, and parenteral. As used herein, the term "parenteral" includes subcutaneous, intravenous, intra-arterial, intraperitoneal, intracardiac, intrathecal, and intramuscular injection, as well as infusion injections.

In instances where parenteral administration is utilized, it may be necessary to employ somewhat bigger oligomers than those described previously, with molecular weights ranging from about 500 to 30K Daltons (e.g., having molecular weights of about 500, 1000, 2000, 2500, 3000, 5000, 7500, 10000, 15000, 20000, 25000, 30000 or even more).

The method of administering may be used to treat any condition that can be remedied or prevented by administration of the particular conjugate. Those of ordinary skill in the art appreciate which conditions a specific conjugate can effectively treat. The actual dose to be administered will vary depend upon the age, weight, and general condition of the subject as well as the severity of the condition being treated, the judgment of the health care professional, and conjugate being administered. Therapeutically effective amounts are known to those skilled in the art and/or are described in the pertinent reference texts and literature. Generally, a therapeutically effective amount will range from about 0.001 mg to 1000 mg, preferably in doses from 0.01 mg/day to 750 mg/day, and more preferably in doses from 0.10 mg/day to 500 mg/day.

The unit dosage of any given conjugate (again, preferably provided as part of a pharmaceutical preparation) can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, administration five times a day, four times a day, three times a day, twice daily, once daily, three times weekly, twice weekly, once weekly, twice monthly, once monthly, and any combination thereof. Once the clinical endpoint has been achieved, dosing of the composition is halted.

### EXPERIMENTAL

All chemical reagents referred to in the appended examples are commercially available unless otherwise indicated. The preparation of PEG-mers is described in, for example, U.S. Patent Application Publication No. 2005/0136031.

All ¹H NMR (nuclear magnetic resonance) data was generated by an NMR spectrometer manufactured by Bruker. A list of certain compounds as well as the source of the compounds is provided below.

### Example 1

### Synthesis of mPEGn-Cinacalcet (n= 1, 2, 3, 4, 5, 6, 7 and so forth)

mPEGn-N-cinacalcet is prepared using a poly(ethylene glycol) bearing a chloroformate. Schematically, the approach followed for this example is shown below.

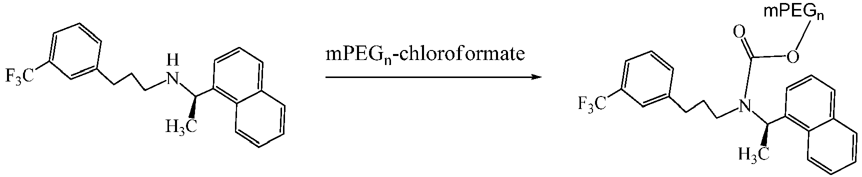

An excess of mPEG₃-chloroformate [Cl-C(O)O-CH₂CH₂O)ₙCH₃] is added to a composition of cinacalcet in the presence of a base at a temperature of 20 °C for two to four hours. The conjugate thus formed is isolated using conventional techniques.

The approach is repeated several times in which each time the approach is repeated, a different mPEGn size [e.g., "n" number of ethylene oxide monomers, wherein n= 1, 2, 4, 5, 6, 7 and so forth] is used.

### Example 2

### Synthesis of mPEGn-Cinacalcet (n= 1, 2, 3, 4, 5, 6, 7 and so forth)

mPEGn-N-cinacalcet is prepared using a poly(ethylene glycol) bearing a leaving group such as a sulphonate (e.g., mesylate or "Ms") or halide. Schematically, the approach followed for this example is shown below.

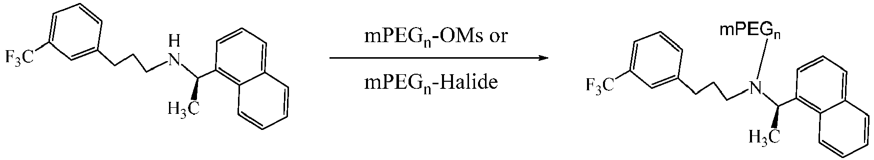

Cinacalcet is dissolved in DMF. To the solution, NaH is added with stirring. After 20 minutes, mPEGₙ-Halide (e.g, mPEGₙ-Br) is added to the solution, which is then stirred overnight at room temperature. Dichloromethane (150 mL) is added and the precipitated solid is collected by filtration. The organic filtrate is washed with water (100 mL × 2) and then dried. The crude product is purified by column chromatography (SiO₂: DCM/Methanol 20:1).

The approach is repeated several times in which each time the approach is repeated, a different mPEGn size [e.g., "n" number of ethylene oxide monomers, wherein n= 1, 2, 4, 5, 6, 7 and so forth] is used.

### Example 3

### Synthesis of mPEGn-Cinacalcet (Relatively Large Oligomer Sizes)

mPEGn-N-cinacalcet wherein the oligomer is relatively large is prepared in accordance with the approach described below.

Briefly, compound **1** is prepared using conventional techniques and reacted with tert-butyl 2-(bis(2-hydroxyethyl)amino)acetate obtained from reacting BICIN with t-BOC anhydride in the presence of the coupling agent *N*-ethyl-*N'*-(3-dimethylaminopropyl)carbodiimide (or EDC) to yield compound **2.**

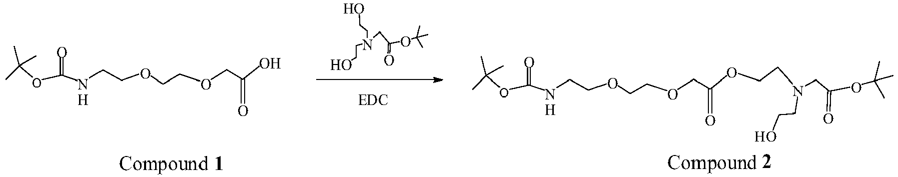

Thereafter, the hydroxyl group of compound **2** is acylated via treatment with reacted with acetyl chloride (AcCl) to provide compound **3.**

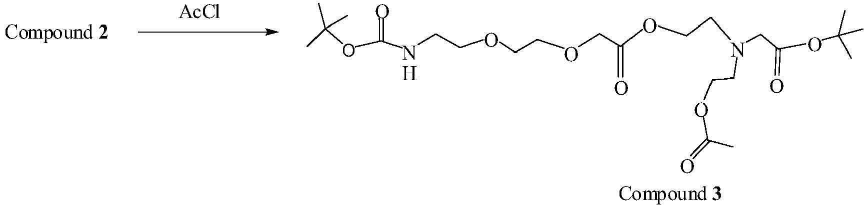

Next, compound **3** is treated with 20% trifluoroacetic acid (TFA) to the TFA salt (compound **4**).

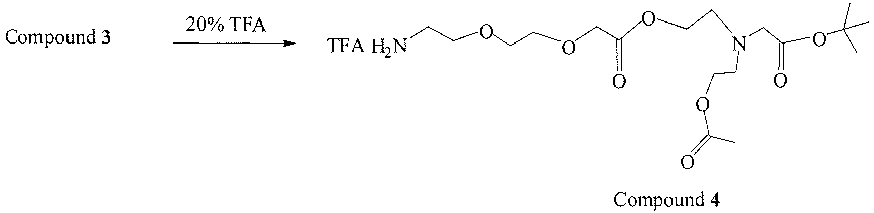

Then, compound **4** is dissolved in a suitable solvent and a succinimidyl carbonate-terminated mPEG reagent (molecular weight of about 2,000 Daltons) is coupled the base form of compound **4** to yield compound **5.** This step can alternatively be carried out with a chloroformate-terminated mPEG reagent in place of the succinimidyl carbonate-terminated mPEG reagent.

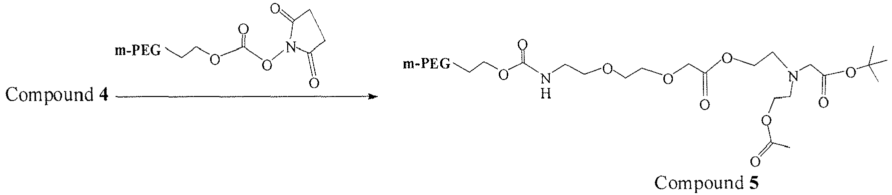

Thereafter, the tert-butyl protecting group within compound **5** is deprotected with 33% TFA to provide a carboxylic acid-containing compound **6.**

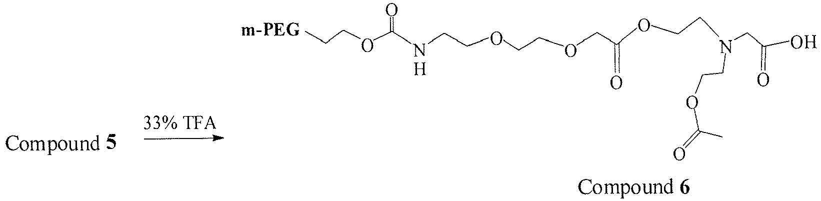

Next, the carboxylic acid of the carboxylic-containing compound **6,** is activated with H-hydroxysuccinimide (NHS) in the presence of a coupling agent to provide compound **7.**

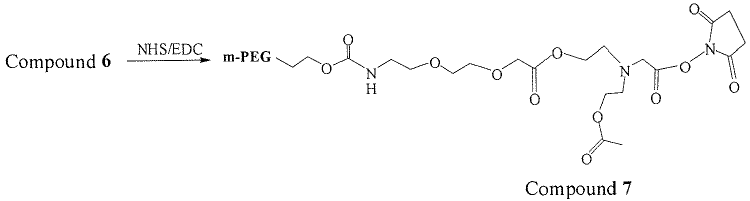

Thereafter, compound **7** is coupled to the secondary amine of cinacalcet to provide compound **8.**

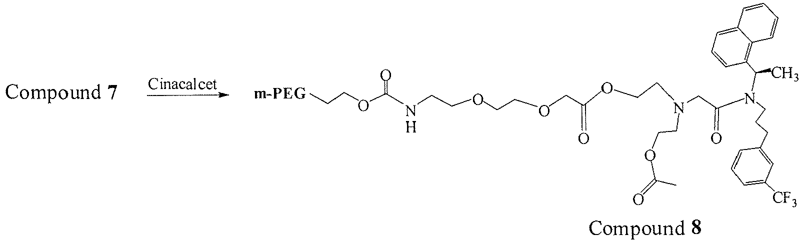

The approach is repeated several times in which each time the approach is repeated, a different mPEG weight (e.g., 5,000 Daltons, 10,000 Daltons, 15,000 Daltons, 20,000 Daltons, 30,000 and 40,000 Daltons) is used.

### Example 4

### Synthesis of mPEGn-Cinacalcet (Relatively Large Oligomer Sizes)

mPEGn-N-cinacalcet wherein the oligomer is relatively large is prepared in accordance with the schematic provided below. In the schematic, compound **9** (in which each mPEG has a molecular weight of about 2,000 Daltons) is the oligomeric reagent that can be prepared as described in U.S. Patent Application Publication No. 2006/0293499 and can be coupled to cinacalcet to provide compound **10.**

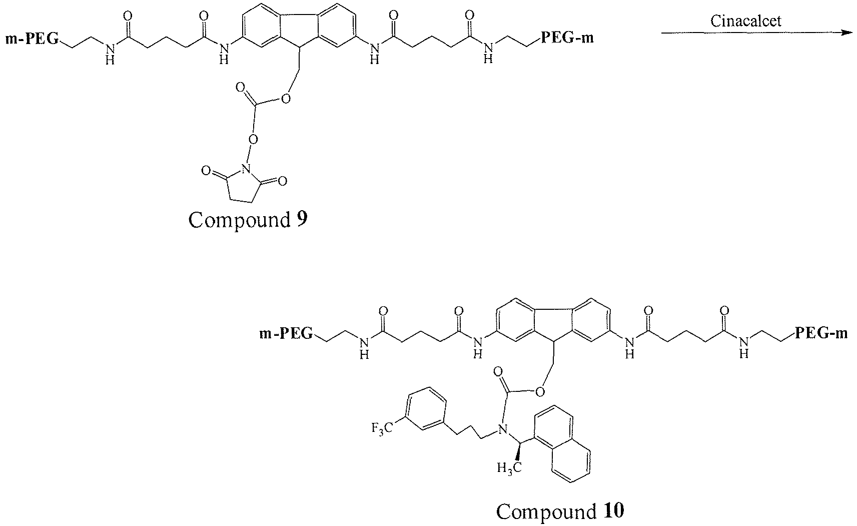

The approach is repeated several times in which each time the approach is repeated, a different mPEG weight (e.g., mPEG weight of 2,500 Daltons to provide a reagent weight of about 5,000 Daltons, mPEG weight of 5,000 Daltons to provide a reagent weight of about 10,000 Daltons, mPEG weight of about 7,500 Daltons to provide a reagent weight of about 15,000 Daltons, mPEG weight of 10,000 Daltons to provide a reagent weight of about 20,000 Daltons, mPEG weight of about 12,500 Daltons to provide a reagent weight of about 25,000 Daltons, mPEG weight of about 15,000 Daltons to provide a reagent weight of about 30,000 Daltons, and mPEG weight of 20,000 Daltons to provide a reagent weight of about 40,000 Daltons) is used.

### Example 5

### Synthesis of mPEG₁₀ₖ-Glycine-Amide-Cinacalcet (15)

Cinacalcet can be linked to an oligomer through a spacer moiety (or "linkage") that contains one or more amino acids. By including one or more amino acids in the linakge, it is possible to affect the overall release rate. For example, a dipeptide can undergo self-cleavage, which represents an extra step in the overall release mechanism and thereby affect the overall release of the active moiety. A compound that includes an amino acid-containing linker between a residue of cinacalcet and an oligomer was prepared following the schematic provided below.

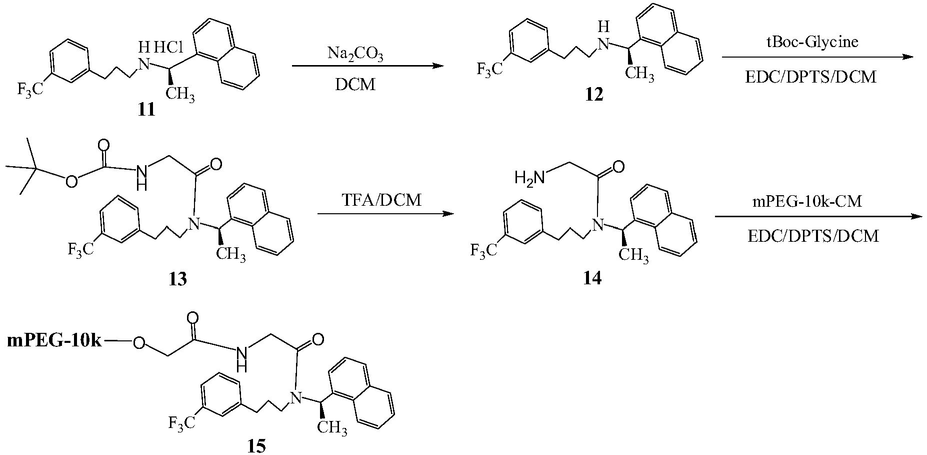

### Preparation of cinacalcet free base (12)

Cinacalcet HCl salt (∼3.16 g, ∼8.02 mmol, **11**) was dissolved in dichloromethane (DCM) and the solution was washed with saturated aqueous Na₂CO₃ solution three times. The dichloromethane solution was dried over Na₂SO₄, filtered, and the solvent removed under reduced pressure to give cinacalcet free base (**12**) as a clear liquid (∼2.75 g, ∼96% yield).

### Synthesis of tBoc-glycine-amide-cinacalcet (13)

Cinacalcet free base (∼1.0 g, ∼2.80 mmol, **12**), *t*Boc-glycine (∼0.98 g, ∼5.60 mmol) and 4-*N,N-*dimethylaminopyridinium *p*-tolunesulfonate (DPTS, ∼0.42 g, ∼1.40 mmol) were dissolved in dichloromethane. *N*-ethyl-*N'*-(3-dimethylaminopropyl)carbodiimide (EDC, ∼1,3 g, ∼8.40 mmol) was then added. The reaction mixture was stirred at room temperature overnight (∼18 hours). The dichloromethane reaction mixture was then washed with 0.2N HCl solution three times. The dichloromethane solution was dried over Na₂SO₄, filtered, and the solvent removed under reduced pressure to give the crude product as a yellow liquid. After chromatography purification, *t*Boc-glycine-amide-cinacalcet (**13**) was obtained as a white solid (∼0.60 g, ∼1.20 mmol, ∼42% isolated yield).

### Synthesis of glycine-amide-cinacalcet (14)

*t*Boc-glycine-amide-cinacalcet (**13**) (∼0.60 g, ∼1.20 mmol) was dissolved in dichloromethane (∼5 mL), and thereafter trifluoroacetic acid (TFA) (∼3 mL) was added and the reaction mixture was stirred at room temperature for four hours. The reaction was monitored by HPLC analysis for completion. The dichloromethane product mixture was washed with saturated aqueous Na₂CO₃ solution twice, dried over Na₂SO₄. After removing all solvents, glycine-amide-cinacalcet (**14**) was obtained as a light-yellow liquid (∼0.50 g, ∼1.20 mmol, quantitative yield).

### Preparation of mPEG₁₀ₖ-glycine-amide-cinacalcet (15)

mPEG₁₀ₖ-CM [CH₃O(CH₂CH₂O)ₙ-CH₂CH₂-OCH₂-COOH, (∼1.2 g, ∼0.12 mmol)], glycine-amide-cinacalcet (**14**) (∼314 mg, ∼0.76 mmol) and 4*-N,N-*dimethylaminopyridinium *p*-tolunesulfonate (DPTS, ∼227 mg, ∼0.76 mmol) were dissolved in dichloromethane. *N*-ethyl-*N'*-(3-dimethylaminopropyl)carbodiimide (EDC, ∼0.40 mL, ∼353 mg, ∼2.27 mmol) was then added. The reaction mixture was stirred at room temperature overnight (∼21 hours). The product mixture was poured into 1:1 isopropanol/diethyl ether with strong stirring, the resulting white precipitate was collected, washed with 1:1 isopropanol/diethyl ether and diethyl ether, and dried in vacuum. mPEG₁₀ₖ-glycine-amide-cinacalcet conjugate (**15**) was obtained as a white solid (∼1.15 g, ∼95% yield). HPLC showed that the product purity was >95% and there was no small molecule impurity. NMR indicated that the cinacalcet substitution was ∼95%.

### Example 6

### Synthesis of a Multi-armed Oligomer-Cinacalcet (16)

A multi-armed oligomer-cinacalcet (also containing an amino acid-containing spacer moiety) was prepared in accordance with the schematic provided below.

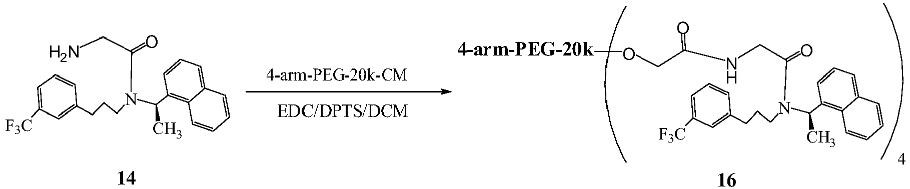

Four-arm-PEG₂₀ₖ-CM [C(CH₂O(CH₂CH₂O)ₙ-CH₂CH₂-OCH₂-COOH)₄ (∼2.0 g, ∼0.10 mmol)], glycine-amide-cinacalcet (**14**) (∼212 mg, ∼0.51 mmol) and 4-*N*,*N-*dimethylaminopyridinium *p*-tolunesulfonate (DPTS, ∼154 mg, ∼0.51 mmol) were dissolved in dichloromethane. *N*-ethyl-*N'*-(3-dimethylaminopropyl)carbodiimide (EDC, ∼0.27 mL, ∼239 mg, ∼1.54 mmol) was then added. The reaction mixture was stirred at room temperature overnight (∼18 hours). The product mixture was poured into 1:1 isopropanol/diethyl ether with strong stirring, the resulting white precipitate was collected, washed with 1:1 isopropanol/diethyl ether and diethyl ether, and dried in vacuum. Four-arm-PEG₂₀ₖ-glycine-amide-cinacalcet conjugate (**16**) was obtained as a white solid (∼1.9 g, ∼95% yield). HPLC analysis showed that the product purity was >95% and there was no small molecule impurity. NMR indicated that the cinacalcet substitution was ∼95%.

### Example 7

### Synthesis of mPEG₁₀ₖ-L-Leucine-Acyloxymethyl-Carbamate-Cinacaleet (20)

A compound that includes an amino acid-containing spacer moiety between a residue of cinacalcet and an oligomer was prepared following the schematic provided below.

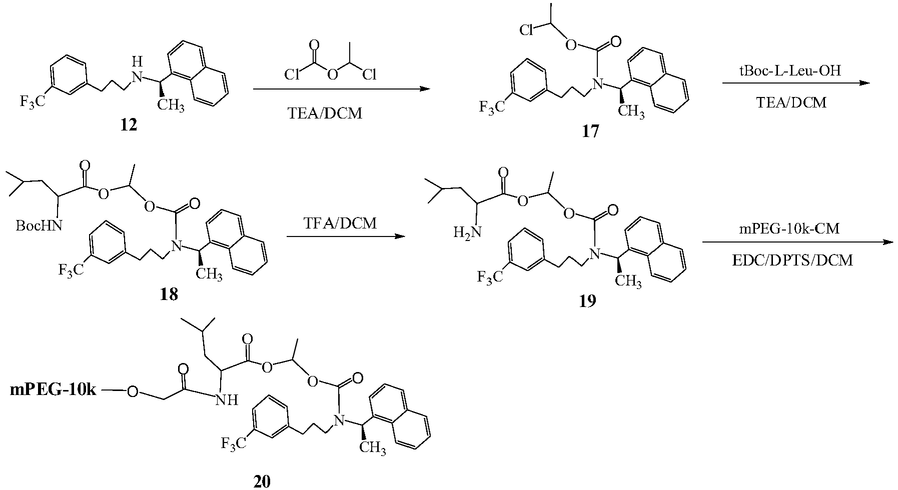

### Synthesis of 1-chloroethyl-carbamate-cinacalcet (17)

To a dichloromethane solution of cinacalcet (∼360 mg, ∼1.0 mmol, **12**) was added 1-chloroethyl chloroformate (∼173 mg, ∼1.20 mmol) and triethylamine (TEA, ∼0.21 mL), ∼153 mg, ∼1.50 mmol), the solution was stirred at room temperature for three hours. The dichloromethane product mixture was then washed with 0.1N HCl/NH₄Cl aqueous solution twice and Na₂CO₃ aqueous solution once. The dichloromethane phase was dried over Na₂SO₄, after solvent evaporation, 1-chloroethyl-carbamate-cinacalcet (**17**) was obtained as a colorless liquid (∼437 mg, ∼94% isolated yield).

### Synthesis of tBoc-L-leucine-acyloxymethyl-carbamate-cinacalcet (18)

To a dichloromethane solution of 1-chloroethyl-carbamate-cinacalcet (**17**) (∼437 mg, ∼0.92 mmol) and *t*Boc-L-leucine (∼871 mg, ∼3.77 mmol) was added triethylamine (TEA, ∼0.39 mL, ∼286 mg, ∼2.83 mmol). The reaction mixture was stirred at room temperature overnight (∼20 hours), then washed with 0.1N HCl/NH₄Cl aqueous solution twice and NaCl aqueous solution once. The crude product was purified by chromatography with hexane/ethyl acetate; *t*Boc-L-leucine-carbamate-cinacalcet (**18**) was obtained as a colorless liquid (∼280 mg, ∼0.43 mmol, ∼45% isolated yield).

### Synthesis of L-leucine-acyloxymethyl-carbamate-cinacalcet (19)

To a dichloromethane solution of *t*Boc-L-leucine-carbamate-cinacalcet (**18**) (∼280 mg, ∼0.43 mmol) was added trifluoroacetic acid (TFA) (∼1.5 mL). The reaction mixture was stirred at room temperature for about one hour. The reaction was monitored by HPLC analysis for completion. The dichloromethane product mixture was washed with 0.1N HCl/NH₄Cl aqueous solution three times. The dichloromethane phase was dried over Na₂SO₄, all solvents were then removed under reduced pressure. L-leucine-acyloxymethyl-carbamate-cinacalcet (**19**) was obtained as a colorless liquid (∼238 mg, quantitative yield).

### Preparation of mPEG₁₀ₖ-L-leucine-acyloxymethyl-carbamate-cinacalcet (20)

mPEG₁₀ₖ-CM (∼1.4 g, ∼0.14 mmol), L-leucine-acyloxymethyl-carbamate-cinacalcet (**19**) (∼115 mg, ∼0.21 mmol) and 4*-N,N-*dimethylaminopyridinium *p-*tolunesulfonate (DPTS, ∼43 mg, ∼0.14 mmol) were dissolved in dichloromethane. *N*-ethyl-*N'*-(3-dimethylaminopropyl) carbodiimide (EDC, -0.20 mL), ∼170 mg, ∼1.10 mmol) was then added. The reaction mixture was stirred at room temperature overnight (∼20 h). The product mixture was poured into 1:1 isopropanol/diethyl ether with strong stirring, the resulting white precipitate was collected, washed with 1:1 isopropanol/diethyl ether and diethyl ether, and dried in vacuum. mPEG₁₀ₖ-L-leucine-acyloxymethyl-carbamate-cinacalcet (**20**) was obtained as a white solid (∼1.3 g, ∼93% yield). HPLC analysis showed that there was no small molecule impurity. NMR indicated that the cinacalcet substitution was ∼80%.

### Example 8

### Synthesis of Multi-armed Oligomer-Cinacalcet (21)

A multi-armed oligomer-cinacalcet (also containing an amino acid-containing spacer moiety) was prepared in accordance with the schematic provided below.

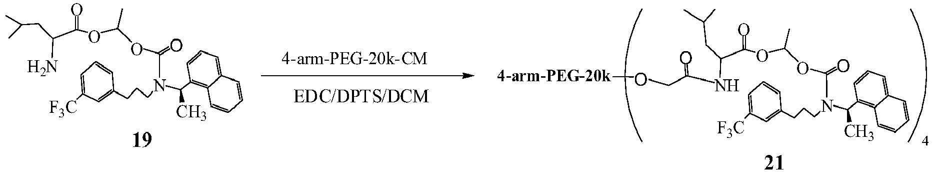

Four-arm-PEG₂₀ₖ-CM (∼760 mg, ∼0.038 mmol), L-leucine-acyloxymethyl-carbamate-cinacalcet (**19**) (∼122 mg, ∼0.22 mmol) and 4*-N,N-*dimethylaminopyridinium *p-*tolunesulfonate (DPTS, ∼65 mg, ∼0.21 mmol) were dissolved in dichloromethane. *N*-ethyl-*N'*-(3-dimethylaminopropyl)carbodiimide (EDC, ∼0.60 mL, ∼520 mg, ∼3.39 mmol) was then added. The reaction mixture was stirred at room temperature overnight (∼22 hours). The product mixture was poured into 1:1 isopropanol/diethyl ether with strong stirring, the resulting white precipitate was collected, washed with 1:1 isopropanol/diethyl ether and diethyl ether, and dried in vacuum. Four-arm-PEG₂₀ₖ-L-leucine-acyloxymethyl-carbamate-cinacalcet (**21**) was obtained as a white solid (∼750 mg, ∼98% yield). HPLC analysis showed that there was no small molecule impurity and the cinacalcet substitution was ∼71%.

### Example 9

### Synthesis of mPEG₁₀ₖ-L-Valine-Acyloxymethyl-Carbamate-Cinacalcet (24)

A compound that includes an amino acid-containing spacer moiety between a residue of cinacalcet and an oligomer was prepared following the schematic provided below.

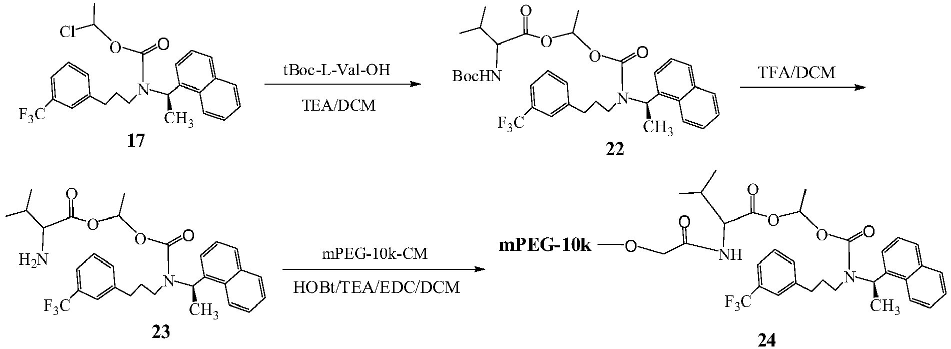

### Synthesis of tBoc-L-valine-acyloxymethyl-carbamate-cinacalcet (22)

To a dichloromethane solution of 1-chloroethyl-carbamate-cinacalcet (**17**) (∼748 mg, ∼1.61 mmol) and *t*Boc-L-valine (∼1.4 g, ∼6.45 mmol) was added triethylamine (TEA, ∼0.67 mL, -489 mg, ∼4.84 mmol). The reaction mixture was stirred at room temperature overnight (∼20 hours), then washed with 0.1N HCl/NH₄Cl aqueous solution twice and NaCl aqueous solution once. The crude product was purified by chromatography with hexane/ethyl acetate; *t*Boc-L-valine-carbamate-cinacalcet (**22**) was obtained as a colorless liquid (∼900 mg, ∼1.40 mmol, ∼87% isolated yield).

### Synthesis of L-valine-acyloxymethyl-carbamate-cinacalcet (23)

To a dichloromethane solution of *t*Boc-L-valine-carbamate-cinacalcet (**22**) (∼900 mg, ∼1.40 mmol) was added trifluoroacetic acid (TFA) (∼2 mL). The reaction mixture was stirred at room temperature for about three hours. The reaction was monitored by HPLC analysis for completion. The dichloromethane product mixture was washed with 0. IN HCl/NH₄Cl aqueous solution three times. The dichloromethane phase was dried over Na₂SO₄. After solvent evaporation, L-valine-acyloxymethyl-carbamate-cinacalcet (**23**) was obtained as a white solid (∼735 mg, ∼97% isolated yield).

### Synthesis of mPEG₁₀ₖ-L-valine-acyloxymethyl-carbamate-cinacalcet (24)

mPEG₁₀ₖ-CM (∼2.3 g, ∼0.23 mmol), L-valine-acyloxymethyl-carbamate-cinacalcet (**23**) (∼180 mg, ∼0.33 mmol), triethylamine (TEA, ∼0.14 mL, ∼100 mg, ∼0.99 mmol) and 1-hydroxybenzotriazole (HOBt, ∼47 mg, ∼0.35 mmol) were dissolved in dichloromethane. *N*-ethyl-*N'*-(3-dimethylaminopropyl)carbodiimide (EDC, ∼0.20 mL, ∼169 mg, ∼1.10 mmol) was then added. The reaction mixture was stirred at room temperature overnight (∼19 hours). The product mixture was poured into 1:1 isopropanol/diethyl ether with strong stirring, the white precipitate was collected, washed with 1:1 isopropanol/diethyl ether and diethyl ether, and dried in vacuum. mPEG₁₀ₖ-L-valine-acyloxymethyl-carbamate-cinacalcet (**24**) was obtained as a white solid (∼2.0 g, ∼87% yield). HPLC analysis showed that there was no small molecule impurity. NMR indicated that the cinacalcet substitution was ∼72%.

### Example 10

### Synthesis of Multi-armed Oligomer-Cinacalcet (25)

A multi-armed oligomer-cinacalcet (also containing an amino acid-containing spacer moiety) was prepared in accordance with the schematic provided below.

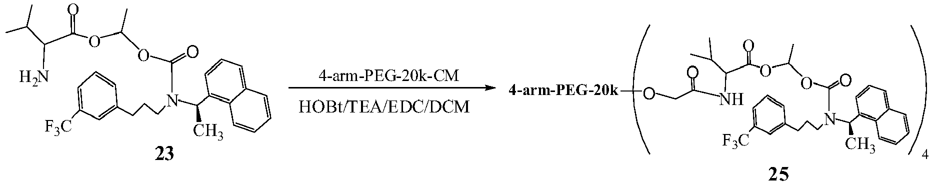

Four-arm-PEG₂₀ₖ-CM (∼1.2 g, ∼0.059 mmol), L-valine-acyloxymethyl-carbamate-cinacalcet (**23**) (∼180 mg, ∼0.33 mmol), triethylamine (TEA, ∼0.14 mL, ∼100 mg, ∼0.99 mmol) and 1-hydroxybenzotriazole (HOBt, ∼47 mg, ∼0.35 mmol) were dissolved in dichloromethane. *N*-ethyl-*N'*-(3-dimethylaminopropyl)carbodiimide (EDC, ∼0.19 mL, ∼154 mg, ∼0.99 mmol) was then added. The reaction mixture was stirred at room emperature overnight (∼24 hours). The product mixture was poured into 1:1 isopropanol/diethyl ether with strong stirring, the white precipitate was collected, washed with 1:1 isopropanol/diethyl ether and diethyl ether, and dried in vacuum. Four-arm-PEG₂₀ₖ-L-valine-acyloxymethyl-carbamate-cinacalcet (**25**) was obtained as a white solid (∼1.0 g, ∼83% yield). HPLC analysis showed that there was no small molecule impurity and NMR indicated that the cinacalcet substitution was ∼80%.

### Example 11

### Synthesis of mPEG₁₀ₖ-D-Valine-Acyloxymethyl-Carbamate-Cinacalcet (28)

A compound that includes an amino acid-containing spacer moiety between a residue of cinacalcet and an oligomer was prepared following the schematic provided below.

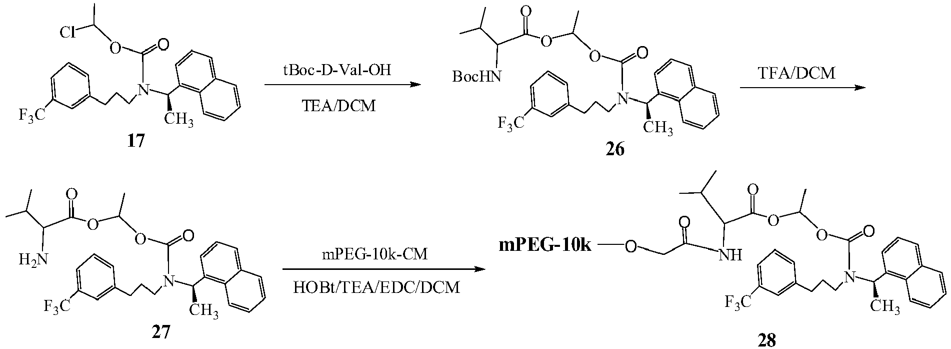

### Synthesis of tBoc-D-valine-acyloxymethyl-carbamate-cinacalcet (26)

To a dichloromethane solution of 1-chloroethyl-carbamate-cinacalcet (**17**) (∼670 mg, ∼1.44 mmol) and *t*Boc-D-valine (∼1.3 g, ∼5.78 mmol) was added triethylamine (TEA, ∼0.60 mL, ∼438 mg, ∼4.33 mmol). The reaction mixture was stirred at room temperature overnight (∼20 hours), then washed with 0.1N HCl/NH₄Cl aqueous solution twice and NaCl aqueous solution once. The crude product was purified by chromatography with hexane/ethyl acetate; *t*Boc-D-valine-carbamate-cinacalcet (**26**) was obtained as a colorless liquid (∼230 mg, ∼0.36 mmol, ∼25% isolated yield).

### Synthesis of D-valine-acyloxymethyl-carbamate-cinacalcet (27)

To a dichloromethane solution of *t*Boc-D-valine-carbamate-cinacalcet (**26**) (∼230 mg, ∼0.36 mmol) was added trifluoroacetic acid (TFA) (∼2 mL). The reaction mixture was stirred at room temperature for ∼4.5 hours. The reaction was monitored by HPLC analysis for completion. The dichloromethane product mixture was washed with 0. IN HCl/NaCl aqueous solution three times. The dichloromethane phase was dried over Na₂SO₄. After solvent evaporation, D-valine-acyloxymethyl-carbamate-cinacalcet (**27**) was obtained as a white solid (∼176 mg, ∼91% isolated yield).

### Synthesis of mPEG₁₀ₖ-D-valine-acyloxymethyl-carbamate-cinacalcet (28)

mPEG₁₀ₖ-CM (∼1.1 g, ∼0.11 mmol), D-valine-acyloxymethyl-carbamate-cinacalcet (**27**) (∼88 mg, ∼0.16 mmol), triethylamine (TEA, ∼0.07 mL, ∼49 mg, ∼0.49 mmol) and 1-hydroxybenzotriazole (HOBt, ∼23 mg, ∼0.17 mmol) were dissolved in dichloromethane. *N-*ethyl-*N*'-(3-dimethylaminopropyl)carbodiimide (EDC, ∼0.09 mL, ∼75 mg, ∼0.49 mmol) was then added. The reaction mixture was stirred at room temperature overnight (∼18 hours). The product mixture was poured into 1:1 isopropanol/diethyl ether with strong stirring, the resulting white precipitate was collected, washed with 1:1 isopropanol/diethyl ether and diethyl ether, and dried in vacuum. mPEG₁₀ₖ-D-valine-acyloxymethyl-carbamate-cinacalcet (**28**) was obtained as a white solid (∼1.1 g, ∼99% yield). HPLC analysis showed that there was no small molecule impurity and the cinacalcet substitution was ∼89%.

### Example 12

### Synthesis of Multi-armed Oligomer-Cinacalcet (29)

A multi-armed oligomer-cinacalcet (also containing an amino acid-containing spacer moiety) was prepared in accordance with the schematic provided below.

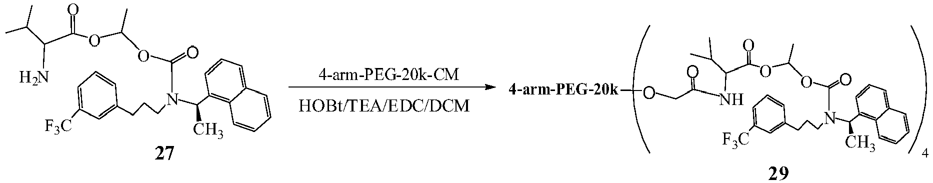

Four-arm-PEG₂₀ₖ-CM (∼582 mg, ∼0.029 mmol), D-valine-acyloxymethyl-carbamate-cinacalcet (**27**) (∼88 mg, ∼0.16 mmol), triethylamine (TEA, ∼0.07 mL, ∼49 mg, ∼0.49 mmol) and 1-hydroxybenzotriazole (HOBt, ∼23 mg, ∼0.17 mmol) were dissolved in dichloromethane. *N-*ethyl-*N*'-(3-dimethylaminopropyl)carbodiimide (EDC, ∼0.09 mL, ∼75 mg, ∼0.49 mmol) was then added. The reaction mixture was stirred at room temperature for 41 hours. The product mixture was poured into 1:1 isopropanol/diethyl ether with strong stirring, the resulting white precipitate was collected, washed with 1:1 isopropanol/diethyl ether and diethyl ether, and dried in vacuum. Four-arm-PEG₂₀ₖ-D-valine-acyloxymethyl-carbamate-cinacalcet (**29**) was obtained as a white solid (∼550 mg, ∼94% yield). HPLC analysis showed that there was no small molecule impurity and NMR indicated that the cinacalcet substitution was ∼70%.

### Example 13

### Hydrolysis of Exemplary Compounds in pH 7.4 PBS Buffer

General procedure: ∼5 mg of a compound of interest was dissolved in ∼10 mL of pH 7.4 PBS buffer which contained ∼12 mM phosphate and ∼140 mM NaCl/KCl. The solution was placed into ten different HPLC vials and the HPLC vials were put into a 37 °C incubator. The samples were analyzed by HPLC at different timepoints. When hydrolysis occurred, HPLC confirmed that cinacalcet was released unaltered. The buffer half-lives t_{1/2} of various PEG-cinacalcet conjugates are listed in Table 2.

**Table 2**

| Hydrolysis of Tested Compounds in pH 7.4 PBS Buffer at 37 °C | |
|---|---|
| **Compound Name** | **t_{1/2} in PBS** |
| mPEG₁₀ₖ-L-leucine-acyloxymethyl-carbamate-cincalcet (**20**), Example 7 | ∼126 hours |
| mPEG₁₀ₖ-D-valine-acyloxymethyl-carbamate-cincalcet (**28**), Example 11 | ∼208 hours |
| mPEG₁₀ₖ-L-valine-acyloxymethyl-carbamate-cincalcet (**24**), Example 9 | ∼317 hours |
| mPEG₁₀ₖ-glycine-amide-cincalcet (**15**), Example 5 | stable |
| 4-Arm-PEG₂₀ₖ-glycine-amide-cincalcet (**16**), Example 6 | stable |

### Example 14

### Hydrolysis of Exemplary Compounds in an In Vivo Rat Study

The hydrolysis rates of exemplary compounds in an in vivo rat study was conducted. Briefly, cinacalcet hydrochloride was administered orally to four male rats to provide a cinacalcet equivalent dose of 10 mg/kg. For this cohort, blood was collected at the following time points: 0 (predose); 1, 2, 4, 6, 9, 12, 24, 48 and 72 hours following administration. Compound (**15**), Compound (**20**), Compound (**16**), Compound (**21**), Compound (**24**), Compound (**25**), Compound (**28**) and Compound (**29**), prepared in accordance with Examples, 5, 7, 6, 8, 9, 10, 11 and 12 respectively, were separately administered intravenously to four male rats to provide a cinacalcet equivalent dose of 1 mg/kg. Parent compound cinacalcet prepared in DMSO at 1mg/kg was separately administrated intravenously to four male rats. For these cohorts, blood was collected at the following time points: 0 (predose), 2 minutes, 10 minutes, 1 hour, 2 hours, 4 hours, 8 hours, 24 hours, 48 hours and 72 hours following administration.

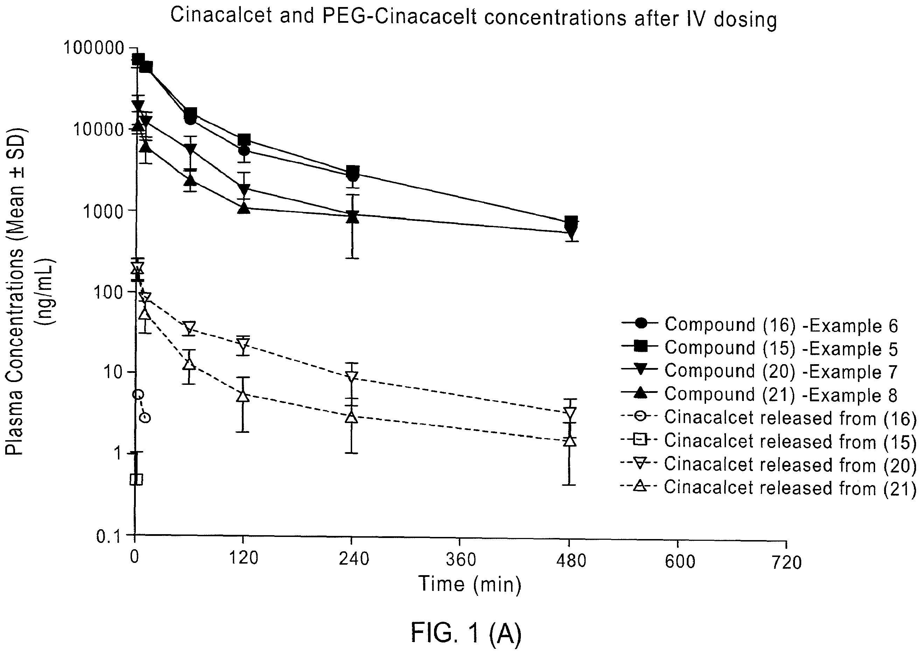

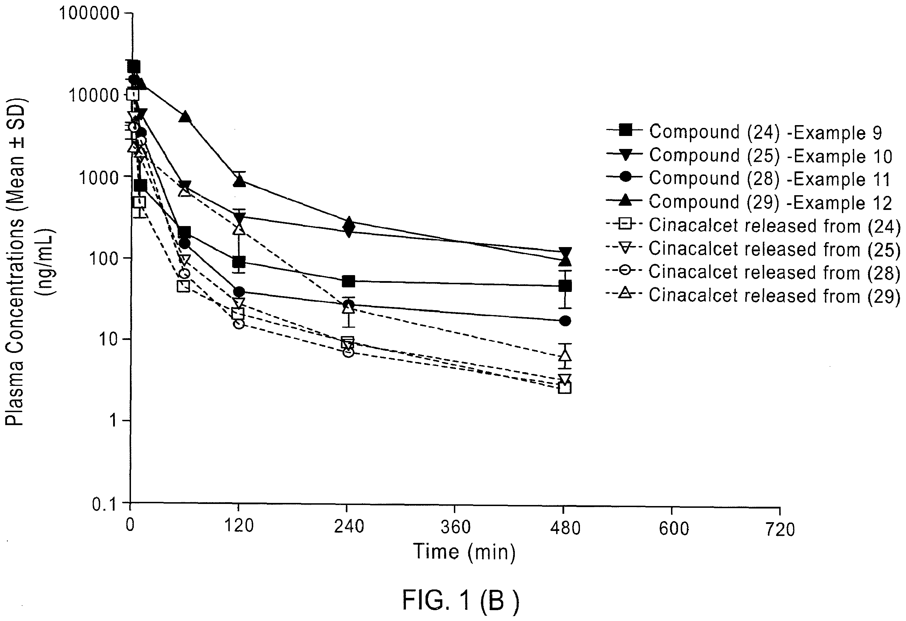

The results are provided in graph form in **FIG. 1A** and **FIG. 1B**, which show the plasma concentration-time profiles (averaged across the four rats) for the tested compounds.

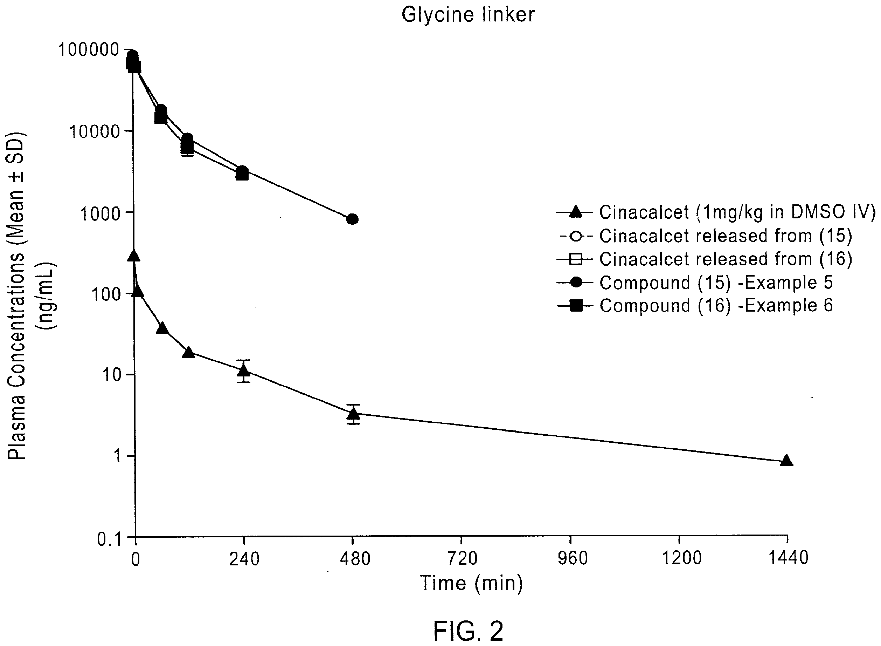

As shown in **FIG. 2****,** comparisons of the plasma concentration-time profiles for released cinacalcet from each Compound (**15**) and Compound (**16**), Examples 5 and 6, respectively, and cinacalcet (unmodified) in DMSO after IV administration. In these two compounds using a glycine-based spacer moiety, only relatively low levels of cinacalcet (<10ng/mL) release could be observed at earlier time points.

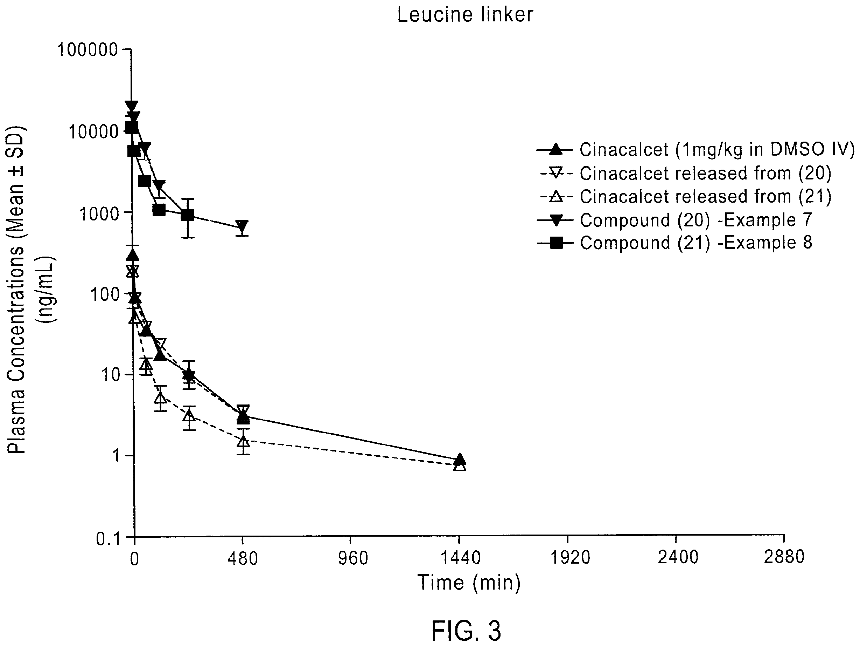

In **FIG. 3****,** the plasma concentration-time profiles of Compound (**20**) and Compound (**21**), and the released cinacalcet from these two compounds were plotted with cinacalcet. In these two Compounds (**20**) and (**21**) using a leucine-based spacer moiety, relatively significant amounts of cinacalcet release were observed.

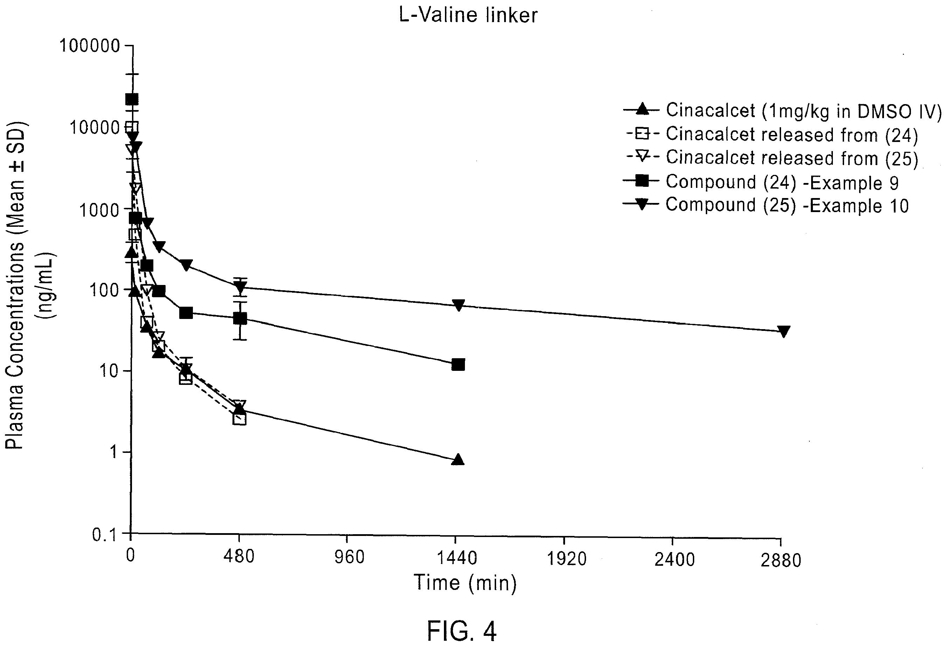

In **FIG. 4****,** the plasma concentration-time profiles of Compound (**24**) and Compound (**25**), and the released cinacalcet from these two compounds were plotted with cinacalcet. In these two Compounds (**24**) and (**25**) using a L-Valine-based spacer moiety, relatively significant amounts of cinacalcet release were observed.

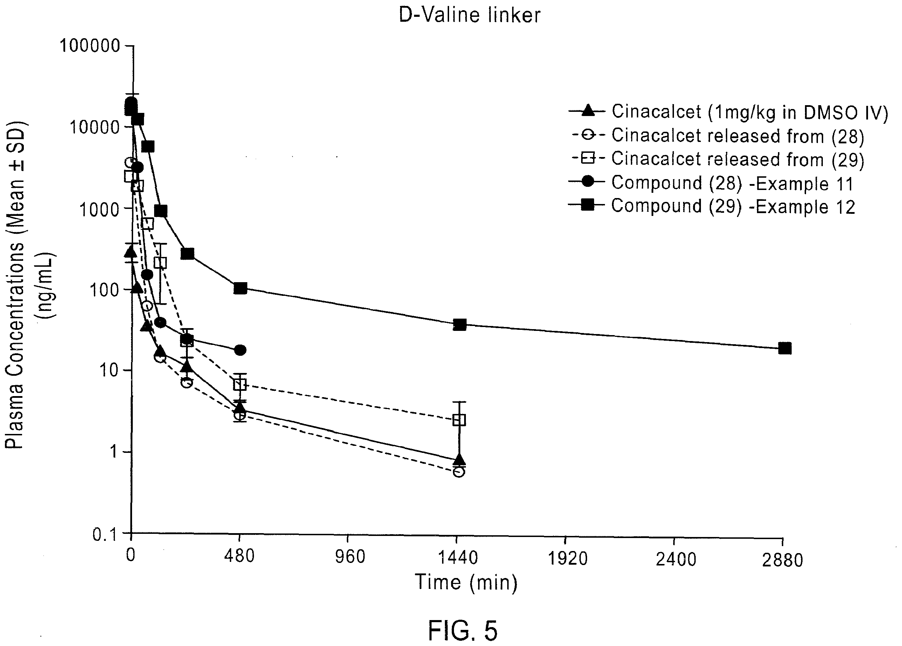

In **FIG. 5****,** the plasma concentration-time profiles of Compound (**28**) and Compound (**29**), and the released cinacalcet from these two compounds were plotted with cinacalcet. In these two Compounds (**28**) and (**29**) using a D-Valine-based spacer moiety, relatively significant amounts of cinacalcet release were observed.

Standard pharmacokinetic parameters were also determined for each of Compound (**20**) and Compound (**21**), Examples 7 and 8, respectively, and cinacalcet (unmodified) and are set forth in Table 3.

**Table 3**

| Pharmacokinetic Parameters of Compound (20), Compound (21) and Cinacalcet | | | | | | |
|---|---|---|---|---|---|---|
| **PK Parameter** | **Test Article** | | | | | |
| | **Compound (20) (Example 7) (1 mg/kg, IV)** | | **Compound 21 (Example 8) (1 mg/kg, IV)** | | **Cinacalcet (10 mg/kg, PO)** | |
| | **Mean** | **SD** | **Mean** | **SD** | **Mean** | **SD** |
| **AUCall (min*ng/mL)** | **11200** | **2210** | **5730** | **2220** | **41400** | **10100** |
| **Clearance (mL/min/kg)** | **100** | **21.1** | **206** | **91.6** | **-** | |
| **Cₘₐₓ (ng/mL)** | **178** | **45.8** | **200** | **58.8** | **97.4** | **14.2** |
| **T_{1/2} (min)** | **139** | **38.8** | **280** | **143** | **179** | **34.8** |
| **Vₛₛ (mL/kg)** | **13600** | **2840** | **28000** | **8930** | **-** | |

In view of this data, it can be concluded that Compounds (**15**) and (**16**) shown substantially no release of cinacalcet after intervenous administration. With respect to Compounds (**20**), (**21**) (**24**), (**25**), (**28**) and (**29**), however, release of cinacalcet continues for up to about 8-12 hours following intravenous administration. In addition, Compound (**20**) showed approximately 2-fold higher exposure and approximately equal Cₘₐₓ compared to Compound (**21**), which had a four-arm oligomer. Compound (**21**), however, appears to have 2-fold longer t_{1/2} and 2-fold higher clearance and volume of distribution (Vₛₛ) compared to Compound (**20**).

Thus, depending on the desired pharmacokinetic profile, the exemplary compounds provide a range of dosing options.

### Example 15

### In Vitro Screen - HEK 293 Cells

Using conventional techniques, human embryonic kidney cells (HEK 293 cells) are engineered to express the human parathyroid calcium sensing receptor (CaR). These cells can detect allosteric activators (calcimimetics) of the CaR using changes in cytoplasmic Ca2+ concentrations as an endpoint (flipper assay). Changes in cytoplasmic Ca2+ concentrations provide a quantitative and functional assessment of CaR activity in these cells. Compounds of the invention are tested using this in vitro model and are shown to function as calicimimetics.

### Example 16

### In Vitro Screen - Dissociated Parathyroid Cells

An in vitro screening based on CaR-dependent regulation of PTH secretion is used to test compounds of the invention. Primary cultures of dissociated bovine parathyroid cells are prepared and aliquots of compounds of the invention are allowed to come into contact with the prepared cells. PTH secretion in the presence of a compound of invention is then compared against a control. Compounds of the invention are tested using this in vitro model and are shown to function as calicimimetics.

### Example 17

### In Vivo Screen - PTH-lowering effects in normal Rats

PEG-Cinacalcet calcimmetic effects were studied in PTH (parathyroid hormone)-lowering experiments in normal rats. Male SD rats (∼350g) were IV dosed with different compounds at 1 mg/kg or orally ("PO") cinacalcet at 10mg/kg. Plasma samples were collected and prepared at different timepoints (up to 72 hours) after drug administration. The plasma PTH levels were quantified by ELISA. Compounds of the invention were tested using this model and are shown to function as calicimimetics.

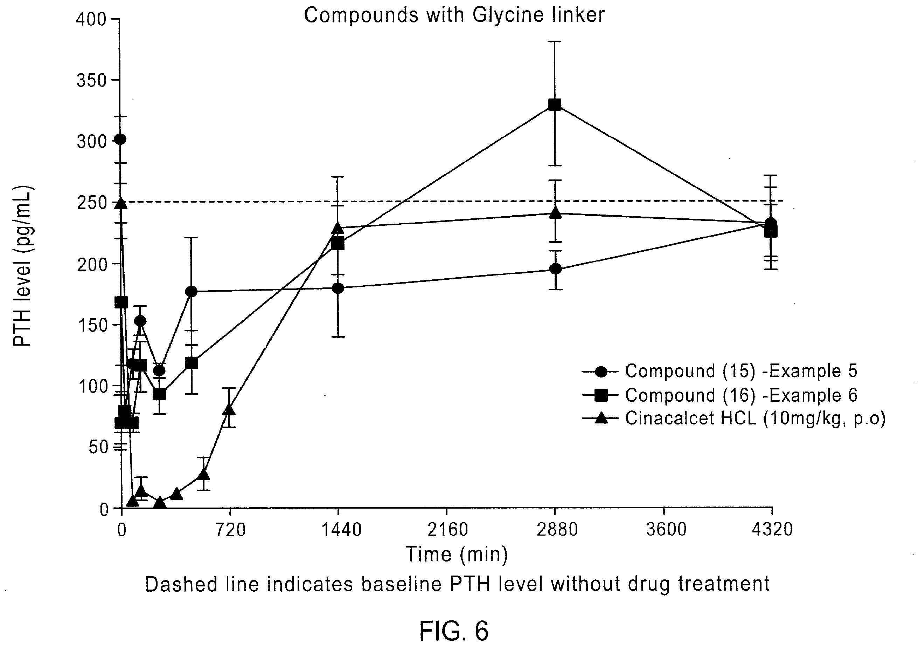

In **FIG. 6** are rat plasma PTH levels after cinacalcet (PO, 10mg/kg) or Compounds (**15**) and (**16**) (IV, 1mg/kg) treatments. At 10mg/kg, parent cinacalcet oral treatment produced a PTH-lowering effect up to ∼12 hours. Although Compound (**15**) and (**16**) did not show significant cinacalcet release in the earlier studies, both compounds showed PTH-lowering effects from 2 minutes to 8 hours after drug administration.

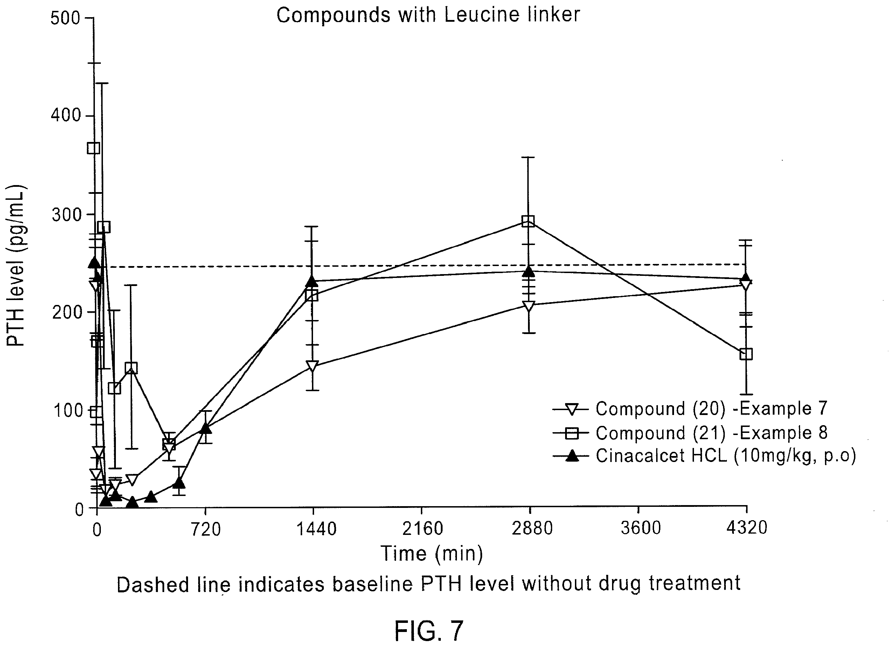

In FIG. 7 are rat plasma PTH levels after cinacalcet (PO, 10mg/kg) or Compounds (20) and (21) (IV, 1mg/kg) treatments. Both compounds showed significant PTH-lowering effects. Compound (20) in particular exhibited prolonged PTH-lowering effects up to ∼24-48 hours.

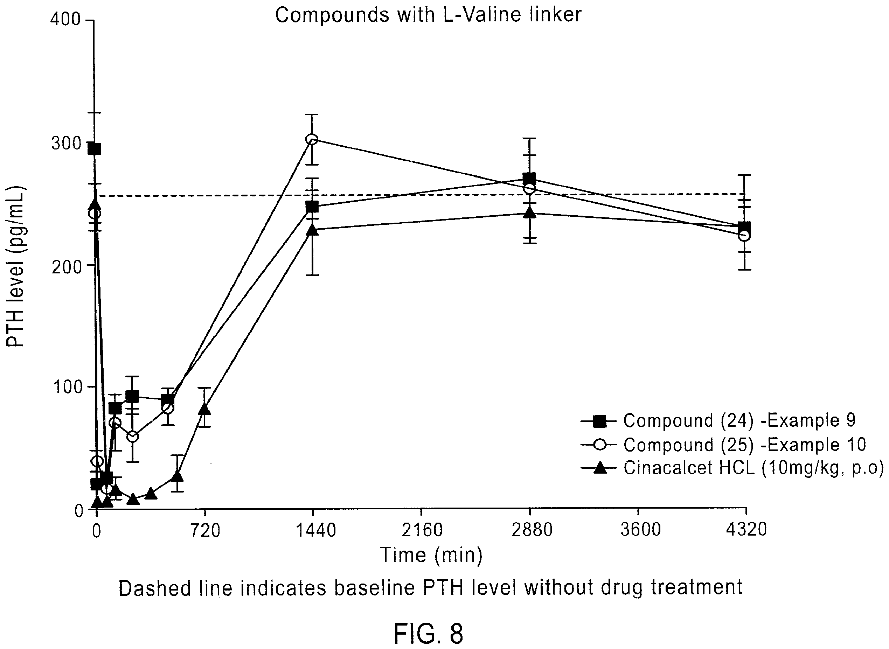

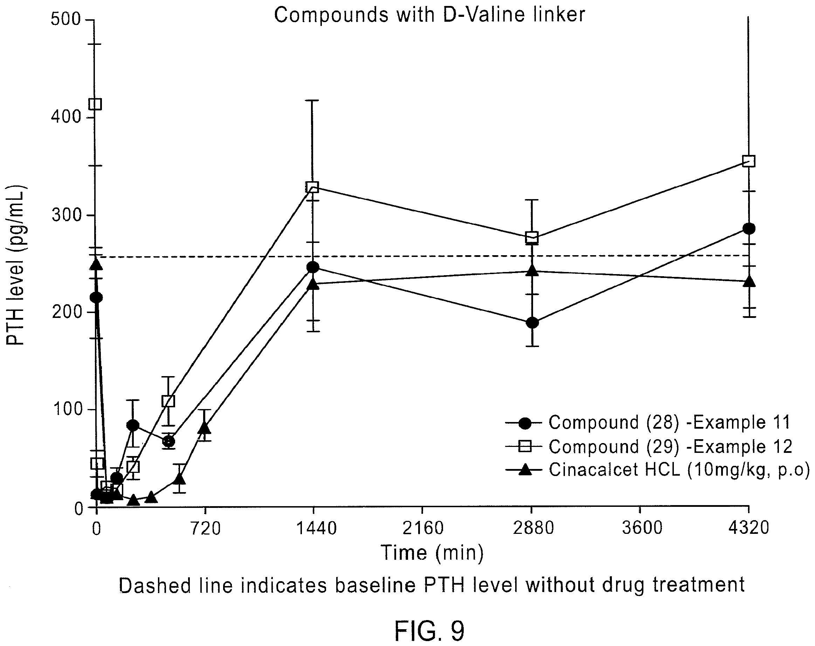

In FIG. 8 are rat plasma PTH levels after cinacalcet (PO, 10mg/kg) or Compounds (24) and (25) (IV, 1mg/kg) treatments. In FIG. 9 are rat plasma PTH levels after cinacalcet (PO, 10mg/kg) or Compounds (28) and (29) (IV, 1mg/kg) treatments. All compounds showed variable PTH-lowering effects, with the animal PTH levels returning to baseline at ∼24hrs.

In summary, PTH-lowering effects of PEG-Cinacalcet compounds were tested in normal rats. The effects were dependent on different linkers and PEG structures being used. Compound (**20**) in particular exhibited prolonged PTH-lowering effect up to ∼24-48 hours in this test, comparing to parent cinacalcet at 10mg/kg PO that only exhibited PTH-lowering effects up to ∼12 hours after dosing.

### Example 18

### In Vivo Screen - Rat Chronic Renal Insufficiency (CRI) Model

The approach set forth in Example 14 is repeated except rats are surgically modified to mimic chronic renal insufficiency through the total removal of one kidney and 2/3 of the other kidney (a 5/6 nephrectomy or "5/6Nx"). Each compound is tested over 6-8 weeks following the 5/6 nephrectomy. Parathyroide levels in normal rats are around 100 pg/ml and in 5/6 Nx rats they increase to 500-1000 pg/ml. Compounds of the invention are tested using this model and are shown to function as calicimimetics, with the potential to be used in chronic kidney disease.

### Example 19

### In Vivo Screen - Rat Parathyroid Hyperplasia Model

5/6 Nx modified rats also mimics parathyroid hyperplasia. As such, compounds of the invention are tested in 5/6 nephrectimized rats and are shown to have potential as decreasing parathyroid hyperplasia (through parathyroid cell proliferation and gland size by removing the parathyroid gland at the end of the study, weighed, sectioned and stained for p21.

### Example 20

### Preparation of Compound 1 (Used in Example 3)

Compound 1 can be prepared using the schematic set forth below, wherein reaction conditions of temperature, amounts, time, pH and solvents will be known to one of ordinary skill in the art upon a review of this schematic and the disclosure provided herein.

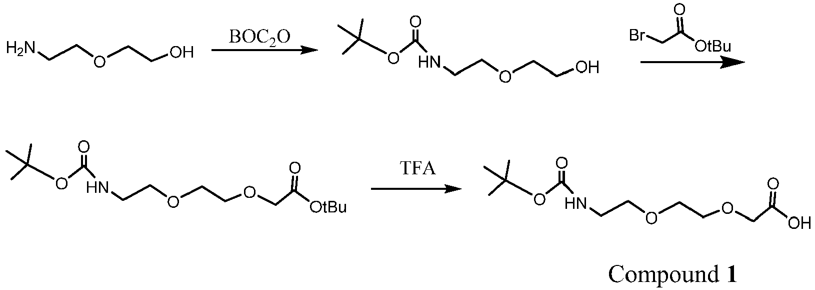

In addition, following this same general approach, H₂N-CH₂CH₂(OCH₂CH₂)ₙ-OH, wherein n is defined as something other than 1 and as otherwise defined herein (e.g., 2, 3, 4, 5, 6, 7, 8, 9, 10 or more) can be used, to provide (CH₃)₃-OC(O)-NH-CH₂CH₂-O(OCH₂CH₂)ₙ-O-CH₂-C(O)OH, wherein n is defined as something other than 1 and as otherwise defined herein (e.g., 2, 3, 4, 5, 6, 7, 8, 9, 10 or more).

## Claims

1. A compound comprising cinacalcet covalently attached via a releasable linkage to a poly(alkylene oxide) oligomer, wherein the linkage contains one or more amino acids.

2. The compound of claim 1, encompassed within the formula: 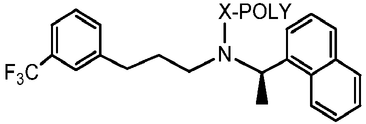 wherein:
X is a spacer moiety; and
POLY is a poly(alkylene oxide) oligomer.

3. The compound of any one of the preceding claims, wherein the poly(alkylene oxide) is a poly(ethylene oxide), or preferably wherein the poly(alkylene oxide) includes an alkoxy or hydroxy end-capping moiety.

4. The compound of any one of the claims 1-3, wherein the poly(alkylene oxide) oligomer has a molecular weight of greater than 2,000 Daltons.

5. The compound of claim 1, wherein the linkage contains a single amino acid, or wherein the linkage contains two amino acids.

6. A composition comprising a compound of claim 1 and optionally, a pharmaceutically acceptable excipient.

7. A compound of the formula 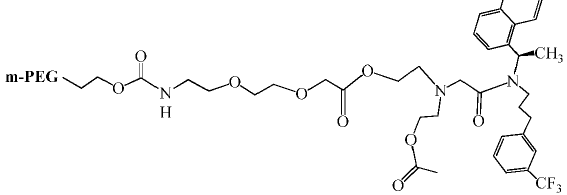 wherein mPEG is a methoxy capped poly(ethylene glycol) polymer having weight average molecular weight selected from about 5,000 Daltons, about 10,000 Daltons, about 15,000 Daltons, about 20,000 Daltons, about 30,000 Daltons, and about 40,000 Daltons.

8. A compound of the formula 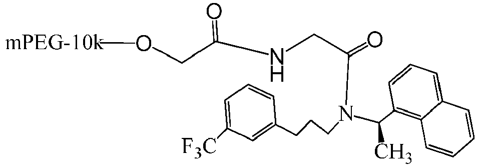 wherein mPEG-10k is a methoxy capped poly(ethylene glycol) polymer having weight-average molecular weight of about 10,000 Daltons.

9. A compound of the formula 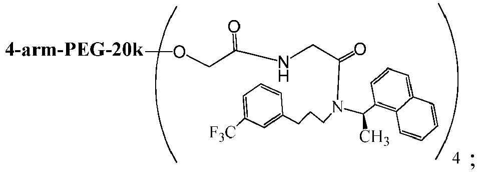 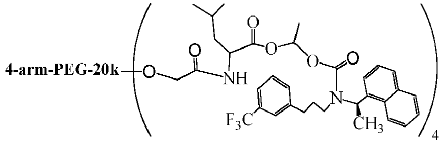 or 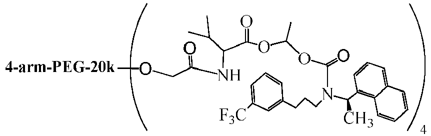 wherein 4-arm-PEG-20k is a four armed poly(ethylene glycol) polymer having a weight-average molecular weight of about 20,000 Daltons.

10. The compound of claim 9, having a formula 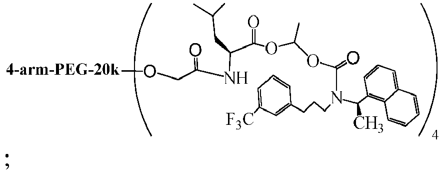 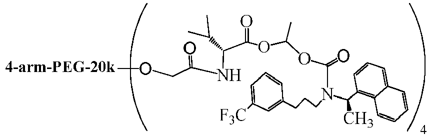 or 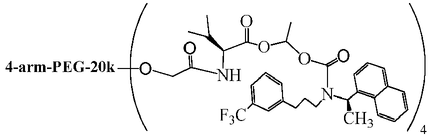 wherein 4-arm-PEG-20k is a four armed poly(ethylene glycol) polymer having a weight-average molecular weight of about 20,000 Daltons.

11. The compound of claim 10, having the formula

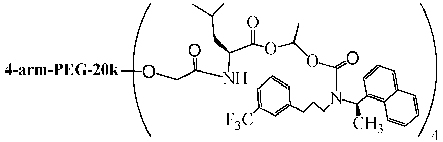

12. A compound of the formula 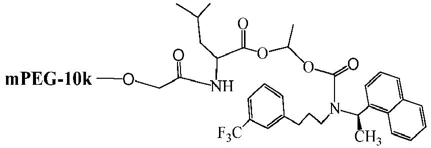 or 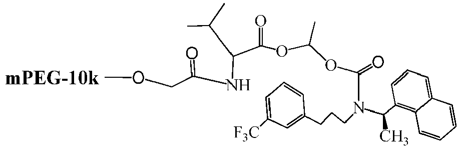 wherein mPEG-10k is a methoxy capped poly(ethylene glycol) polymer having weight-average molecular weight of about 10,000 Daltons.

13. The compound of claim 12, having the formula 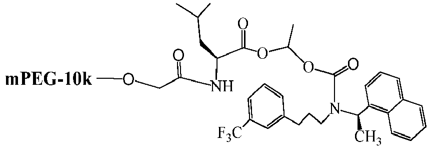 wherein mPEG-10k is a methoxy capped poly(ethylene glycol) polymer having weight-average molecular weight of about 10,000 Daltons.

14. A composition comprising a compound of any one of claims 7-13 and, optionally, a pharmaceutically acceptable excipient.

15. A compound of any one of claims 7-13 for use as a calcimimetic.

## Patentansprüche

1. Verbindung, die Cinacalcet umfasst, das kovalent mittels einer lösbaren Verknüpfung an ein Poly(alkylenoxid)-Oligomer angebracht ist, wobei die Verknüpfung eine oder mehr Aminosäuren enthält.

2. Verbindung nach Anspruch 1, umfasst durch die folgende Formel: 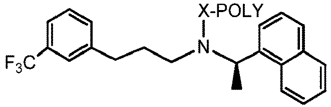 wobei:
X ein Spacerrest ist; und
POLY ein Poly(alkylenoxid)-Oligomer ist.

3. Verbindung nach einem der vorhergehenden Ansprüche, wobei das Poly(alkylenoxid) ein Poly(ethylenoxid) ist, oder bevorzugt, wobei das Poly(ethylenoxid) einen Alkoxy-oder Hydroxyendgruppenrest umfasst.

4. Verbindung nach einem der Ansprüche 1-3, wobei das Poly(alkylenoxid)-Oligomer ein Molekulargewicht von größer als 2000 Dalton aufweist.

5. Verbindung nach Anspruch 1, wobei die Verknüpfung eine einzelne Aminosäure enthält, oder wobei die Verknüpfung zwei Aminosäuren enthält.

6. Zusammensetzung, die eine Verbindung nach Anspruch 1 und gegebenenfalls einen pharmazeutisch akzeptablen Hilfsstoff umfasst.

7. Verbindung der Formel 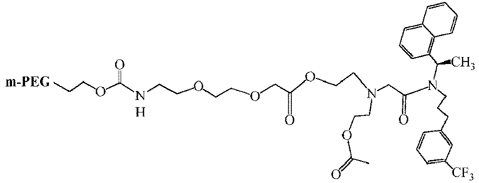 wobei mPEG ein Poly(ethylenglycol)-Polymer mit einer Methoxyendgruppe ist, das ein gewichtsmittleres Molekulargewicht ausgewählt aus ungefähr 5000 Dalton, ungefähr 10000 Dalton, ungefähr 15000 Dalton, ungefähr 20000 Dalton, ungefähr 30000 Dalton und ungefähr 40000 Dalton aufweist.

8. Verbindung der Formel 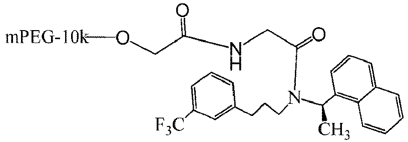 wobei mPEG-10k ein Poly(ethylenglycol)-Polymer mit einer Methoxyendgruppe ist, das ein gewichtsmittleres Molekulargewicht von ungefähr 10000 Dalton aufweist.

9. Verbindung der Formel 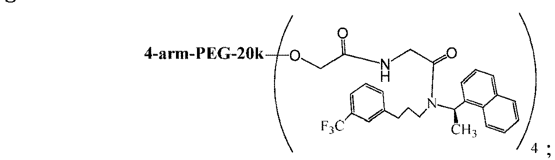 oder 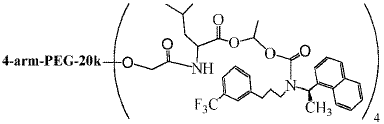 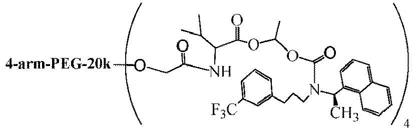 wobei 4-arm-PEG-20k ein vierarmiges Poly(ethylenglycol)-Polymer ist, das ein gewichtsmittleres Molekulargewicht von ungefähr 20000 Dalton aufweist.

10. Verbindung nach Anspruch 9, die ein Formel; 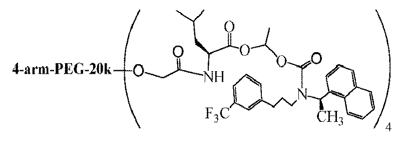 oder 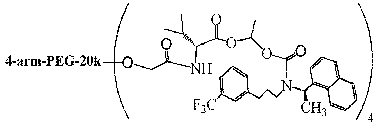 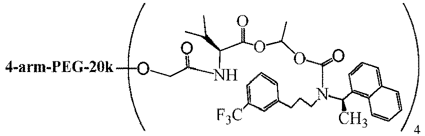 aufweist, wobei 4-arm-PEG-20k ein vierarmiges Poly(ethylenglycol)-Polymer ist, das ein gewichtsmittleres Molekulargewicht von ungefähr 20000 Dalton aufweist.

11. Verbindung nach Anspruch 10, die die Formel 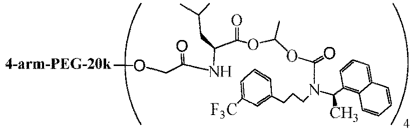 aufweist.

12. Verbindung der Formel 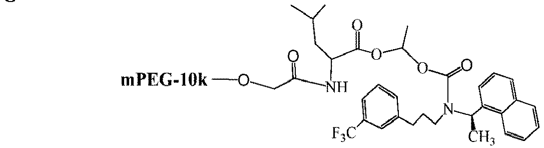 oder 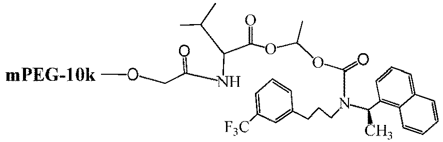 wobei mPEG-10k ein Poly(ethylenglycol)-Polymer mit einer Methoxyendgruppe ist, das ein gewichtsmittleres Molekulargewicht von ungefähr 10000 Dalton aufweist.

13. Verbindung nach Anspruch 12, die die Formel 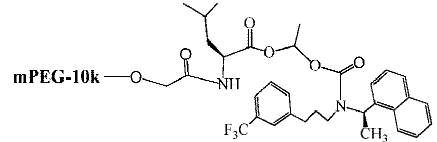 aufweist, wobei mPEG-10k ein Poly(ethylenglycol)-Polymer mit einer Methoxyendgruppe ist, das ein gewichtsmittleres Molekulargewicht von ungefähr 10000 Dalton aufweist.

14. Zusammensetzung, die eine Verbindung nach einem der Ansprüche 7-13 und gegebenenfalls ein pharmazeutisch akzeptablen Hilfsstoff umfasst.

15. Verbindung nach einem der Ansprüche 7-13 zur Verwendung als ein Kalzimimetikum.

## Revendications

1. Composé comprenant du cinacalcet lié de manière covalente par l'intermédiaire d'une liaison pouvant être rompue à un oligomère de poly(oxyde d'alkylène), la liaison contenant un ou plusieurs acides aminés.

2. Composé de la revendication 1, représenté par la formule : 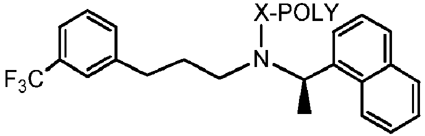 dans laquelle :
X représente un groupement espaceur ; et
POLY représente un oligomère de poly(oxyde d'alkylène).

3. Composé de l'une quelconque des revendications précédentes, dans lequel le poly(oxyde d'alkylène) est un poly(oxyde d'éthylène), ou de préférence dans lequel le poly(oxyde d'alkylène) comporte un groupement alkoxy ou hydroxy de coiffage d'extrémité.

4. Composé de l'une quelconque des revendications 1 à 3, dans lequel l'oligomère de poly(oxyde d'alkylène) a un poids moléculaire supérieur à 2000 Daltons.

5. Composé de la revendication 1, dans lequel la liaison contient un seul acide aminé, ou dans lequel la liaison contient deux acides aminés.

6. Composition comprenant un composé de la revendication 1 et facultativement, un excipient pharmaceutiquement acceptable.

7. Composé répondant à la formule 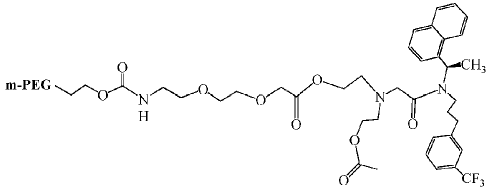 dans laquelle mPEG représente un polymère de poly-(éthylèneglycol) coiffé par un groupe méthoxy ayant un poids moléculaire moyen en poids choisi entre environ 5000 Daltons, environ 10 000 Daltons, environ 15 000 Daltons, environ 20 000 Daltons, environ 30 000 Daltons et environ 40 000 Daltons.

8. Composé répondant à la formule 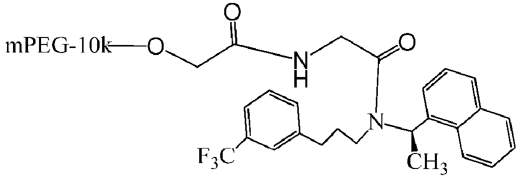 dans laquelle mPEG-10k représente un polymère de poly-(éthylèneglycol) coiffé par un groupe méthoxy ayant un poids moléculaire moyen en poids d'environ 10 000 Daltons.

9. Composé répondant à la formule 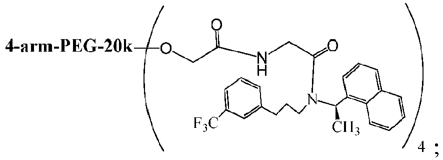 ou 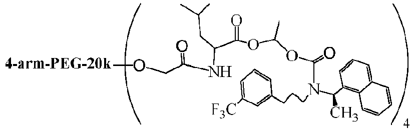 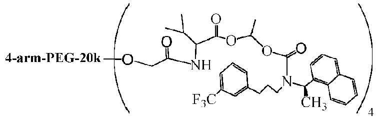 dans laquelle 4-arm-PEG-20k représente un polymère de poly(éthylèneglycol) à quatre bras ayant un poids moléculaire moyen en poids d'environ 20 000 Daltons.

10. Composé de la revendication 9, répondant à la formule 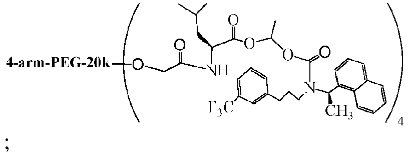 ou 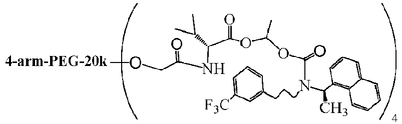 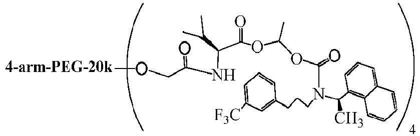 dans laquelle 4-arm-PEG-20k représente un polymère de poly(éthylèneglycol) à quatre bras ayant un poids moléculaire moyen en poids d'environ 20 000 Daltons.

11. Composé de la revendication 10, répondant à la formule

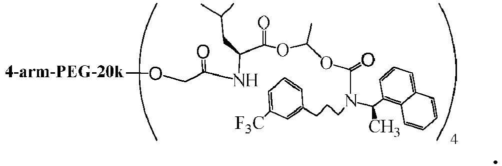

12. Composé répondant à formule 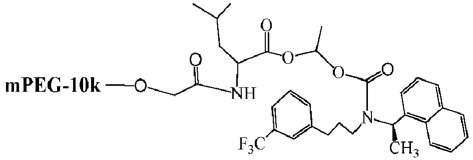 ou 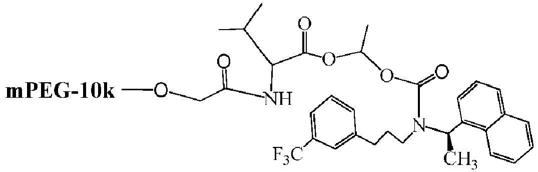 dans laquelle mPEG-10k représente un polymère de poly(éthylèneglycol) coiffé par un groupe méthoxy ayant un poids moléculaire moyen en poids d'environ 10 000 Daltons.

13. Composé de la revendication 12, répondant à la formule 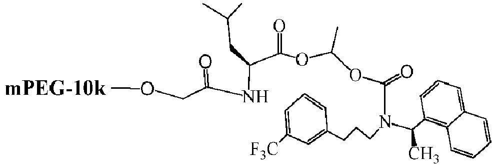 dans laquelle mPEG-10k représente un polymère de poly(éthylèneglycol) coiffé par un groupe méthoxy ayant un poids moléculaire moyen en poids d'environ 10 000 Daltons.

14. Composition comprenant un composé de l'une quelconque des revendications 7 à 13 et, facultativement, un excipient pharmaceutiquement acceptable.

15. Composé de l'une quelconque des revendications 7 à 13 destiné à être utilisé en tant qu'agent calcimimétique.
